# EUROPÄISCHE PATENTANMELDUNG

(11) **EP 3 338 780 A1**
(43) Veröffentlichungstag der Anmeldung: **27.06.2018**
(21) Anmeldenummer: 16205339.1
(22) Anmeldetag: 20.12.2016
(51) Int. Cl.: A61K 31/513, A61P 15/00

(54) **VERWENDUNG VON CHYMASEINHIBITOREN ZUR BEHANDLUNG VON ENDOMETRIOSE, POST-OPERATIVER FIBROSE UND ERKRANKUNGEN DIE DURCH FIBROSEBILDUNG GEKENNZEICHNET SIND**

(71) Anmelder: Bayer Pharma Aktiengesellschaft, 13353 Berlin (DE)
(72) Erfinder: Peters, Michaele, 10405 Berlin (DE)
(74) Vertreter: BIP Patents

(57) **Zusammenfassung**

Die vorliegende Anmeldung betrifft die Verwendung von bicyclisch-substituierten Uracil-Derivaten, allein oder in Kombinationen mit anderen Wirkstoffen zur Behandlung und/oder Prophylaxe von Krankheiten, insbesondere zur Behandlung und/oder Prophylaxe von entzündlichen und fibrotischen Erkrankungen, zur Behandlung von Endometriose, von Endometriose-assoziierter Fibrose, von Adenomyose und von mit einer Endometrioseerkrankung assoziierten Schmerzen sowie von postoperativer peritonealer Fibrose und Adhäsionsbildung.

## Beschreibung

Die vorliegende Anmeldung betrifft die Verwendung von bicyclisch-substituierten Uracil-Derivaten, allein oder in Kombinationen mit anderen Wirkstoffen zur Behandlung und/oder Prophylaxe von Krankheiten, insbesondere zur Behandlung und/oder Prophylaxe von entzündlichen und fibrotischen Erkrankungen, zur Behandlung von Endometriose, von Endometriose-assoziierter Fibrose, von Adenomyose und von mit einer Endometrioseerkrankung assoziierten Schmerzen sowie von postoperativer peritonealer Fibrose und Adhäsionsbildung.

In vielen Erkrankungen führt lokale Entzündung zu der Bildung von Fibrose. Als Fibrose wird eine krankhafte Vermehrung des Bindegewebes in menschlichen und tierischen Geweben und Organen bezeichnet, dessen Hauptbestandteil Kollagenfasern sind. Die Ausbildung von Fibrosen, wenn sie zwei unterschiedliche Gewebe oder Organe betrifft, diese miteinander verbindet führt zu Adhäsionen die die Funktion der Organe einschränken können. Insbesondere zur Therapie und/oder Prophylaxe solcher Erkrankungen mit lokaler Entzündung und Fibrosebildung sind die hier beschriebenen Chymase Inhibitoren geeignet da sie einen entscheidenden Pathomechanismus von Mastzellen verhindern.

Mastzellen sind Schlüsselzellen der Induktion und Regulation von Entzündungsprozessen. Allen Mastzellen sind unabhängig von Spezies und Lokalisation charakteristische Merkmale gemeinsam:
- Sie exprimieren die membranständigen Rezeptoren FcεRI (für IgE) und Kit (für stem cell factor, SCF).
- Sie produzieren metachromatische zytoplasmatische Granula, die präformierte Mediatoren wie Histamin, Proteasen und Proteoglykane enthalten.
Humane Mastzellen lassen sich in Tryptase- und/oder Chymase-positive Populationen aufteilen.

Aktivierte Mastzellen spielen eine wichtige Rolle bei der Wundheilung und in inflammatorischen Prozessen, wie z.B. bei der Entstehung von Fibrosen an Wunden, Angiogenese und kardialem Remodeling (Miyazaki et al., Pharmacol. Ther. 112 (2006), 668-676; Shiota et al., J. Hypertens. 21 (2003), 1823-1825). Chymase-positive Mastzellen können auch eine wichtige Rolle in dem vaskulären Remodeling der Atemwege bei Asthma spielen. Eine erhöhte Anzahl der Mastzellen wurde in endobronchialen Biopsien von Asthmapatienten gefunden (Zanini et al., J. Allergy Clin. Immunol. 120 (2007), 329-333).

Neben einer Funktion bei Asthma und kardiovaskulären Erkrankungen werden aktivierten, Chymase-positiven Mastzellen wichtige pathophysiologische Funktionen in anderen inflammatorischen Erkrankungen zugeschrieben (Heuston et al., Br. J. Pharmacol. 167 (2012) 732-740), ein Beispiel ist die Endometriose (Hart, Int J Inflam. 26 (2015) 1-10).

Der Pathomechanismus der Chymase positiven Mastzellen in solchen Erkrankungen ist auf die Freisetzung aktiver Chymase zurückzuführen. Nach einer Aktivierung der Mastzellen wird Chymase in die extrazelluläre Matrix freigesetzt und aktiviert. Chymase ist eine Chymotrypsin-ähnliche Serinprotease, die als makromolekularer Komplex mit Heparin-Proteoglykanen in sekretorischen Vesikeln von Mastzellen gespeichert wird. Als Protease spaltet Chymase eine Reihe von Substraten. Chymase ist an verschiedenen physiologischen Prozessen beteiligt (Aktivierung von proinflammatorischen Zytokinen, Rekrutierung von Immunzellen, Fibrose und Adhäsionsbildung (Pejler et al. 95. 2007, Adv Immunol. 167-255). Diese Prozesse haben alle eine pathologische Bedeutung, auch in der Endometriose und der postoperativen Fibrosebildung.
Chymase führt zum Abbau von extrazellulären Matrixproteinen, wie Fibronektin, Prokollagen und Vitronektin, und zum Abreißen von fokalen Adhäsionen. Sie bewirkt die Aktivierung und Freisetzung von TGFß (transforming growth factor beta) aus seiner latenten Form, das eine wichtige Rolle in der Entstehung von peritonealen Adhäsionen und Fibrose nach Operationen und auch in der Endometriose spielt. Die Wirkung von Chymase führt zu einer Freisetzung und Aktivierung des Zytokins Interleukin 1 beta (IL-1β), welches ein Hauptmediator von proinflammatorischen Reaktionen ist (Lopez-Castejon et al., Cytokine Growth Factor Rev. 22 (2011) 189 - 95). Durch die Aktivierung von Interleukin 1 beta kommt es zu einer Sensitivierung von Nervenzellen und damit der Entstehung von Schmerzempfinden. Auch andere proinflammatorische Zytokine werden durch die Wirkung von Chymase aktiviert. Dadurch kommt es zu einer Rekrutierung von weiteren Immunzellen, die die Inflammation lokal im Gewebe erhöhen. Chymase ist somit an einer Verstärkung der Entzündung, der Entstehung von Schmerzen, der Rekrutierung von Immunzellen und der Bildung von Adhäsionen beteiligt. Chymase wird als ein mögliches pharmakologisches Target zur Behandlung von inflammatorischen Erkrankungen beschrieben (Heuston et al., Br. J. Pharmacol. 167 (2012) 732-740).

Die Möglichkeit, Chymase-Inhibitoren für die Therapie unterschiedlicher Krankheiten zu verwenden, wurde in zahlreichen tierexperimentellen Studien nachgewiesen. So konnte durch Inhibition der Chymase die Bildung von peritonealen Adhäsionen in einem Hamster Modell reduziert werden (Okamoto et al, J. Surg. Res., 107 (2002) 219-222 und Fertil and Steril. 77(2002) 1044-1048). Es konnte auch gezeigt werden, dass durch Inhibition der Chymase Entzündungen in Pfotenödem-Modellen reduziert werden (Greco et al., J Med Chem 50 (2007) 1727-1730; Maryanoff et al, Am J Respir Crit Care Med 181 (2010) 247 -253). Beta-Ketophosphonat, ein Chymaseinhibitor, blockiert die IL-1β Generierung und verhindert die Einwanderung von infammatorischen Zellen in einem Peritonitis Modell (Garavilla et al., J Biol Chem 280 (2005) 18001-7).

Endometriose ist eine inflammatorische Erkrankung (Lousse JC et al., Front Biosci. 4 (2012) 23-40), die durch das Wachstum von endometrialem Gewebe (Läsionen) außerhalb des Cavum Uteri in der Bauchhöhle und dem Vorkommen von peripheren Nervenendigungen in der Nähe von Läsionen (McKinnon et al., TrendsEndocrinol& Metab. 26 (2015) 1-10) charakterisiert ist. Charakteristisch für die Endometriose ist die Bildung einer inflammatorischen Umgebung in der Bauchhöhle, die oft mit einer Fibrose und der dadurch hervorgerufenen Bildung von Adhäsionen einhergeht. Die Hauptsymptome dieser Erkrankung sind Schmerzen.
Die Inflammation wird als ein möglicher wichtiger Pathomechanismus der Endometriose und als eine Ursache des mit Endometriose assoziiertem Schmerzes gesehen (Laux-Biehlmann et al., Trends in Pharmacol Sci. 36 (2015) 270-276).

Endometriose wird standardmäßig durch Hormon- und/oder Schmerztherapie behandelt. Als nicht-medikamentöse Therapie steht die operative Entfernung der endometriotischen Läsionen und Fibrosen zur Verfügung.

Hormonelle Therapien bewirken eine Absenkung des Östrogenspiegels, der als wesentlicher Faktor bei der Entstehung und Erhaltung der Endometriose angesehen wird. Die wichtigsten Wirkstoffe in der hormonellen Therapie der Endometriose sind:
- Gestagene wie Medroxyprogesteronacetat, Levonorgestrel, Dydrogesteron und Dienogest. Die Verabreichung kann sowohl oral (beispielsweise Dienogest in Visanne®) als auch lokal (beispielsweise Levonorgestrel in einem intrauterinen System) erfolgen.
- einphasige Östrogen-Gestagen-Kombinationspräparate wie beispielsweise in oralen Antikonzeptiva (Pille) enthalten, führen bei kontinuierlicher Einnahme zu einer Rückbildung der Gebärmutterschleimhaut und Schmerzminderung.
- Gonadotropin-Releasing-Hormon-(GnRH)-Analoga blockieren die Hypothalamus-Hypophysen-Achse und senken die Östrogenspiegel in einen Bereich wie er normalerweise bei Frauen nach den Wechseljahren zu erwarten ist. Dies sind Beschwerden, wie Hitzewallungen, Schlafstörungen, trockene Scheide, Knochenschwund und Stimmungsschwankungen.

Hormonelle Antikonzeptiva (Pille) und intrauterine Levonorgestrel-freisetzende Systeme (Intrauterinpessar) sind für die Behandlung der Endometriose in Deutschland nicht zugelassen. Ihre therapeutische Anwendung erfolgt insofern außerhalb des zugelassenen Indikationsbereiches ("off label").

Sämtliche beschriebenen hormonellen Therapien führen nach dem Absetzen zu einem wieder Auftreten von Symptomen und müssten daher über einen sehr langen Zeitraum durchgeführt werden was nicht bei allen möglich ist. So erzeugen einige hormonellen Therapien wie z.B. GnRH Analoga einen vorübergehenden Zustand wie in den Wechseljahren mit den entsprechenden Begleiterscheinungen. So leiden die Patientinnen an Hitzewallungen, Schlafstörungen, Stimmungsschwankungen, Libidoverlust und Knochenschwund (Osteoporose) was eine Therapiedauer von mehr als 6 Monaten schwierig macht.
Die dauerhafte Therapie mit Gestagenen oder der Pille führt bei vielen Frauen durch den Einfluss auf den körpereigenen hormonellen Zyklus zu Nebenwirkungen wie Kopfschmerzen und psychischen Veränderungen. Außerdem werden viele Patientinnen resistent gegenüber einer hormonellen Therapie mit der Pille oder Gestagenen.

Zur Behandlung von Schmerzen können Schmerzmittel, wie beispielsweise Acetylsalicylsäure, Ibuprofen oder Diclofenac angewendet werden. Eine Schmerztherapie kann auf Grund der Nebenwirkungen auf die Nieren und den Magen, aber auch das Kardiovaskuläre System, immer nur kurzzeitig und in enger Absprache mit dem Arzt angewendet werden.

Bei einer operativen Therapie, beispielsweise mit elektrischem Strom, Laser oder dem Skalpell wird versucht, die Endometrioseherde möglichst komplett zu entfernen. Der Eingriff erfolgt überwiegend im Rahmen einer Bauchspiegelung, manchmal ist ein Bauchschnitt notwendig. Bei ausgeprägten Befunden müssen manchmal auch Teile des Eierstocks oder des Eileiters entfernt werden. Nach einer operativen Entfernung der Endometriose kommt es häufig längerfristig wieder zum Auftreten der Erkrankung.

Der Bedarf an neuen Therapien zur Behandlung der Endometriose, die chronisch angewendet werden können und frei von den oben genannten Nebenwirkungen sind ist daher immens.

Auch bei der Endometriose sind Mastzellen und insbesondere Chymase aus Mastzellen an der Pathophysiologie beteiligt: Ein Vergleich von endometriotischen Läsionen mit dem Endometrium zeigt eine stark erhöhte Anzahl von aktivierten Mastzellen in endometriotischen Läsionen (Sugamata et al., J. Reprod. Immunol. 53 (2005) 120-125); Fujiwara et al, J. of Reprod. Immunol. 51 (2004) 341-344). Eine starke Erhöhung der Anzahl aktivierter und Chymase-exprimierender Mastzellen wird in der Nähe von peritonealen Nerven und in tief infiltrierenden Läsionen in der Endometriose beschrieben (Anaf et al., Fertil.Steril. 86 (2006) 1336-1343). Auch in peritonealen Adhäsionen wird eine Erhöhung von Mastzellen gezeigt (Xu et al., Ann. Surg. 236 (2001)593-601).
Mastzellen wird eine wichtige pathophysiologische Rolle in der Endometriose zugeschrieben (Kirchhoff et al., Expert Opin Ther Targets, 16 (2012) 237-241; Hart, Int J Inflam. 26 (2015) 1-10).
Mastzellen in endometriotischen Läsionen sind Chymase positiv (Anaf et al., Fertil.Steril. 86, 2006, 1336-1343). Dabei ist die mastzellspezifische Chymaseexpression in endometriotischen Läsionen stark erhöht im Vergleich zur mastzellspezifischen Expression von Chymase im Endometrium (Paula et al., J Mol Histol.46 (2015) 33-43).

Postoperative peritoneale Fibrose und Adhäsionen werden ausgelöst durch peritoneale Operationen z.B. Laparoskopieen oder Laparotomiene. Bei diesen Operationen kommt es häufig zu Verletzungen von Gewebe an Organen und daraus resultierenden lokalen Entzündungsherden. Diese lokalen Entzündungen führen im Peritoneum zu der Bildung von Fibrose und der Verbindung von Organen durch fibrotisches Gewebe d.h. Adhäsionen. Adhäsionen von Organen im Bauchraum können die Funktion der Organe beeinträchtigen und so unter anderem zu Suberfertilität, zu Darmverschluss und zu Chronischen Bauchschmerzen führen.
Derzeit werden zur Prävention solcher Nebenwirkungen von Peritonealen Operationen physische Barrieren bei der Operation mit eingesetzt um die Adhäsionsausbildung zu reduzieren. Dies ist aber nur direkt bei der Operation möglich und wirkt daher nur kurzfristig. Für eine längerfristige Prävention von postoperativen Fibrosen und Adhäsionen können Medikamente wie zum Beispiel Kortikosteroide oder andere starke Immunsuppressiva, die das Immunsystem und die Aktivität der Bindegewebszellen hemmen eingesetzt werden. Die Verwendung dieser Medikamente ist allerdings mit starken Nebenwirkungen wie das sogenannte Cushing-Syndrom, einem Blutdruckanstieg, einer Blutzuckererhöhung und einer gesteigerten Infektanfälligkeit assoziiert.
Der Bedarf an neuen Therapien zur Prävention von postoperativen Fibrosen und Adhäsionen, die chronisch angewendet werden können und frei von den oben genannten Nebenwirkungen sind ist daher immens.

Auch hier ist die von Mastzellen gebildete Chymase von besonderer Bedeutung für die Pathophysiologie. So konnte durch Inhibition der Chymase die Bildung von peritonealen Adhäsionen in einem Hamster Modell reduziert werden (Okamoto et al, J. Surg. Res., 107 (2002) 219-222 und Fertil and Steril. 77(2002) 1044-1048).

Die Aufgabe der vorliegenden Erfindung liegt darin, Möglichkeiten zur Behandlung und Prävention von entzündlichen Erkrankungen und chronischen Schmerzerkrankungen des kleinen Beckens, wie z.B. Endometriose und/oder sekudärer Dysmenohrö und/oder postoperativer Fibrose und Adhäsionen zur Verfügung zu stellen, die gegenüber den aus dem Stand der Technik bekannten Nachteilen sich auszeichnen durch chronische Anwendbarkeit und ohne Einfluss auf den hormonellen Zyklus der Frau und ohne Einfluss auf die Infektanfälligkeit.
Insbesondere sollen Möglichkeiten zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen gefunden werden.

Die vorliegende Erfindung betrifft die Verwendung von Verbindungen der allgemeinen Formel (I) 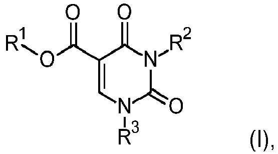 in welcher
- R¹: für Wasserstoff oder (C₁-C₄)-Alkyl steht,
- R²: für eine Gruppe der Formel 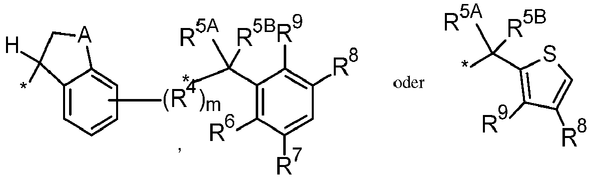 steht, wobei
* für die Anknüpfungsstelle an das Uracil-Stickstoffatom steht,
A für -CH₂-, -CH₂-CH₂-, -O-CH₂-## oder Sauerstoff steht,
worin
## für die Anknüpfstelle an den Phenyl-Ring steht,
m für eine Zahl 0, 1 oder 2 steht,
R⁴ für Halogen, Difluormethyl, Trifluormethyl, (C₁-C₄)-Alkyl, Difluormethoxy, Trifluormethoxy oder (C₁-C₄)-Alkoxy steht,
R^{5A} für Wasserstoff oder Deuterium steht,
R^{5B} für Wasserstoff, Deuterium oder (C₁-C₄)-Alkyl steht,
R⁶ für Wasserstoff oder Fluor steht,
R⁷ für Wasserstoff oder Fluor steht,
R⁸ für Halogen, Difluormethyl, Trifluormethyl, (C₁-C₄)-Alkyl oder Nitro steht,
R⁹ für Wasserstoff, Halogen, Difluormethyl, Trifluormethyl, (C₁-C₄)-Alkyl, Nitro oder (C₁-C₄)-Alkylthio steht,
- R³: für eine Gruppe der Formel 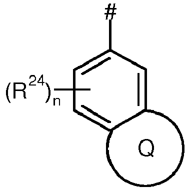 steht, wobei
# für die Anknüpfungsstelle an das Uracil-Stickstoffatom steht,
der Ring Q für 5- bis 7-gliedriges Heterocyclyl oder 5- oder 6-gliedriges Heteroaryl steht,
   worin 5- bis 7-gliedriges Heterocyclyl und 5- oder 6-gliedriges Heteroaryl mit 1 bis 4 Substituenten unabhängig voneinander ausgewählt aus der Gruppe Halogen, Difluormethyl, Trifluormethyl, Trideuteromethyl, (C₁-C₆)-Alkyl, (C₃-C₇)-Cycloalkyl, Oxo, Hydroxy, (C₁-C₄)-Alkylcarbonyl, (C₁-C₄)-Alkoxycarbonyl, Aminocarbonyl und (C₁-C₄)-Alkylsulfonyl substituiert sein können,
   worin (C₁-C₆)-Alkyl und (C₃-C₇)-Cycloalkyl ihrerseits mit 1 bis 3 Substituenten unabhängig voneinander ausgewählt aus der Gruppe Halogen, Cyano, Trifluormethyl, (C₃-C₇)-Cycloalkyl, Hydroxy, (C₁-C₄)-Alkoxy und 4- bis 7-gliedriges Heterocyclyl substituiert sein können,
   und
   worin zwei an ein Kohlenstoffatom von 5- bis 7-gliedriges Heterocyclyl und 5- oder 6-gliedriges Heteroaryl gebundene (C₁-C₆)-Alkyl-Reste zusammen mit dem Kohlenstoffatom, an das sie gebunden sind, einen 3- bis 6-gliedrigen Carbocyclus bilden können,
R²⁴ für Halogen, (C₁-C₄)-Alkyl oder (C₁-C₄)-Alkoxy steht,
n für eine Zahl 0, 1, 2 oder 3 steht
sowie ihrer Salze, Solvate und Solvate der Salze zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.

In einer weiteren Ausführungsform der Erfindung sind die Verbindungen geeignet zur Verwendung bei der Behandlung der Endometriose, der Endometriose-assoziierten Fibrose, der Adenomyose und des mit einer Endometrioseerkrankung assoziierten Schmerzes, von Endometriose-assozierten Adhäsionen und Fibrosen, von Endometriose-assoziierten Symptomen wie Dysmenorrhö, Dyspareunie, Dysurie oder Dyschezie sowie von von Endometriose-assoziierter Subfertilität.

In einer weiteren Ausführungsform der Erfindung sind die Verbindungen geeignet zur Verwendung bei der Behandlung und Vorbeugung von postoperativen Adhäsionen in der Bauchhöhle und zur Vorbeugung von adhäsionsbedingter Subfertilität.

In einer weiteren Ausführungsform der Erfindung sind die Verbindungen geeignet zur Behandlung von Morbus Dupuytren (einer Erkrankung des Bindegewebes der Handinnenflächen) und bei der Behandlung und/oder Prophylaxe von chronischen Schmerzen im kleinen Becken.

Im Rahmen der vorliegenden Erfindung haben die Substituenten, soweit nicht anders spezifiziert, die folgende Bedeutung:

Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl, 2-Ethylbutyl, 1,4-Dimethylpentyl, 4,4-Dimethylpentyl und 1,4,4-Trimethylpentyl.

Cycloalkyl steht in Rahmen der Erfindung für einen monocyclischen, gesättigten Alkylrest mit 3 bis 7 Kohlenstoffatomen. Beispielhaft und vorzugsweise seien genannt: Cyclopropyl, Cyclobutyl, Cyclopentyl, Cyclohexyl und Cycloheptyl.

Alkylcarbonyl steht im Rahmen der Erfindung für einen linearen oder verzweigten Alkylrest mit 1 bis 4 Kohlenstoffatomen und einer in 1-Position angebundenen Carbonylgruppe. Beispielhaft und vorzugsweise seien genannt: Methylcarbonyl, Ethylcarbonyl, n-Propylcarbonyl, iso-Propylcarbonyl, n-Butylcarbonyl, iso-Butylcarbonyl und tert.-Butylcarbonyl.

Alkoxy steht im Rahmen der Erfindung für einen linearen oder verzweigten Alkoxyrest 1 bis 4 Kohlenstoffatomen. Beispielhaft und vorzugsweise seien genannt: Methoxy, Ethoxy, *n*-Propoxy, Isopropoxy, *n*-Butoxy und *tert*.-Butoxy.

Alkoxycarbonyl stehen im Rahmen der Erfindung für einen linearen oder verzweigten Alkoxyrest mit 1 bis 4 Kohlenstoffatomen und einer am Sauerstoff angebundenen Carbonylgruppe. Beispielhaft und vorzugsweise seien genannt: Methoxycarbonyl, Ethoxycarbonyl, n-Propoxycarbonyl, Isopropoxycarbonyl und *tert*.-Butoxycarbonyl.

Alkylthio steht im Rahmen der Erfindung für einen linearen oder verzweigten Alkylrest mit 1 bis 4 Kohlenstoffatomen, der über ein Schwefelatom gebunden ist. Beispielhaft und vorzugsweise seien genannt: Methylthio, Ethylthio, *n*-Propylthio, Isopropylthio, 1-Methylpropylthio, *n*-Butylthio, *iso*-Butylthio und *tert*.-Butylthio.

Alkylsulfonyl steht in Rahmen der Erfindung für einen linearen oder verzweigten Alkylrest mit 1 bis 4 Kohlenstoffatomen, der über eine Sulfonylgruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.

4- bis 7-gliedriges Heterocyclyl steht im Rahmen der Erfindung für einen monocyclischen, gesättigten Heterocyclus mit insgesamt 4 bis 7 Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Beispielhaft seien genannt: Azetidinyl, Oxetanyl, Pyrrolidinyl, Pyrazolidinyl, Tetrahydrofuranyl, Piperidinyl, Piperazinyl, Tetrahydropyranyl, Morpholinyl, Thiomorpholinyl. Bevorzugt sind: Azetidinyl, Oxetanyl, Pyrrolidinyl, Tetrahydrofuranyl, Piperidinyl, Piperazinyl, Tetrahydropyranyl und Morpholinyl.

5- bis 7 gliedriges Heterocyclyl steht im Rahmen der Erfindung für einen teilweise ungesättigten Heterocyclus mit insgesamt 5 bis 7 Ringatomen, der 1 bis 3 Ring-Heteroatome aus der Reihe N, O, S und/oder SO₂ enthält und an den Phenylring in R³ annelliert ist. Beispielhaft seien genannt: Dihydropyrrolyl, Dihydroimidazolyl, Dihydrothiazoldioxid, Dihydrooxazolyl, Dihydropyridyl, Tetrahydropyrazinyl und Dihydrooxazinyl.

Heteroaryl steht im Rahmen der Erfindung für einen monocyclischen aromatischen Heterocyclus (Heteroaromaten) mit insgesamt 5 oder 6 Ringatomen, der bis zu drei gleiche oder verschiedene Ring-Heteroatome aus der Reihe N, O und/oder S enthält und an den Phenylring in R³ annelliert ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl. Bevorzugt sind Pyrazolyl, Imidazolyl, Thiazolyl und Triazolyl.

Halogen schließt im Rahmen der Erfindung Fluor, Chlor, Brom und Iod ein. Bevorzugt sind Chlor oder Fluor.

Eine Oxo-Gruppe steht im Rahmen der Erfindung für ein Sauerstoffatom, das über eine Doppelbindung an ein Kohlenstoff- oder Schwefelatom gebunden ist.

In den Formeln der Gruppe, für die R² und R³ stehen können, steht der Endpunkt der Linie, an dem ein Zeichen * bzw. # bzw. ## steht, nicht für ein Kohlenstoffatom beziehungsweise eine CH₂-Gruppe, sondern ist Bestandteil der Bindung zu dem jeweils bezeichneten Atom, an das R² bzw. R³ gebunden sind.

Wenn Reste in den Verbindungen der allgemeinen Formel (I) substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit einem oder zwei gleichen oder verschiedenen Substituenten ist bevorzugt. Ganz besonders bevorzugt ist die Substitution mit einem Substituenten.

Im Sinne der vorliegenden Erfindung wird unter Endometriose eine inflammatorische Erkrankung (Lousse JC et al., Front Biosci 4 (2012) 23-40] verstanden, die durch das Wachstum von endometrialem Gewebe (Läsionen) außerhalb des Cavum Uteri in der Bauchhöhle charakterisiert ist.

Im Sinne der vorliegenden Erfindung wird unter Fibrose eine krankhafte Neubildung von Bindegewebsfasern verstanden. Grundsätzlich können alle Gewebe und Organe betroffen sein.

Im Sinne der vorliegenden Erfindung wird unter einer Adhäsion eine physische Verbindung zwischen Organen verstanden, die im gesunden Menschen nicht direkt miteinander verbunden sind. Grundsätzlich können alle Gewebe und Organe betroffen sein.

Im Sinne der vorliegenden Erfindung wird unter postoperativen Adhäsion die durch physischen Eingriff in das Peritoneum wie einer Laparoskopie oder Laparotomie z.B. im Rahmen einer Endometriose Behandlung oder einer Tubenligation, entstehenden physische Verbindung zwischen Organen verstanden, die im gesunden Menschen nicht direkt miteinander verbunden sind. Grundsätzlich können alle Gewebe und Organe betroffen sein.

Im Sinne der vorliegenden Erfindung wird unter Adenomyose (Adenomyosis uteri) eine Erkrankung verstanden, bei der sich Endometriosezellen in der Gebärmuttermuskulatur (Myometrium) einnisten und sich damit in der mittleren Schicht der Gebärmutterwand ansiedeln. Die gewanderten Endometriosezellen befinden sich also zusätzlich zu den Zellen der innen gelegenen Gebärmutterschleimhaut auch in der darüber liegenden Gebärmuttermuskulatur. Dies kann zu einer Vergrößerung und Verdickung der Gebärmutter führen. Diese Vergrößerungen der Gebärmutter können an einer einzelnen Stelle oder über die gesamte Muskulatur der Gebärmutter verteilt auftreten.

Im Sinne der vorliegenden Erfindung wird unter sekundärer Dysmenorrhö der sekundäre Regelschmerz, ausgelöst durch organische Veränderungen oder Erkrankungen wie z.B. Endometriose oder Adhäsionen, verstanden.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Bevorzugt im Rahmen der vorliegenden Erfindung ist die Verwendung von Verbindungen der Formel (I), in welcher
- R¹: für Wasserstoff, Methyl oder Ethyl steht,
- R²: für eine Gruppe der Formel 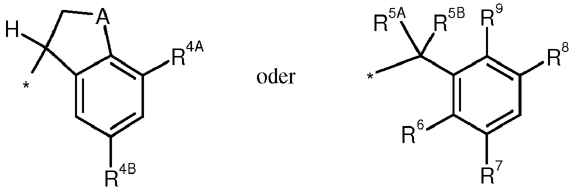 steht, wobei
* für die Anknüpfungsstelle an das Uracil-Stickstoffatom steht,
A für -CH₂- oder Sauerstoff steht,
R^{4A} für Wasserstoff, Fluor, Chlor, Trifluormethyl oder Methyl steht,
R^{4B} für Wasserstoff, Fluor, Chlor, Trifluormethyl oder Methyl steht, mit der Maßgabe, dass mindestens einer der Reste R^{4A} und R^{4B} von Wasserstoff verschieden ist,
R^{5A} für Wasserstoff steht,
R^{5B} für Wasserstoff steht,
R⁶ für Wasserstoff steht,
R⁷ für Wasserstoff steht,
R⁸ für Fluor, Chlor, Difluormethyl, Trifluormethyl oder Methyl steht,
R⁹ für Fluor, Chlor, Difluormethyl, Trifluormethyl oder Methyl steht,
- R³: für eine Gruppe der Formel 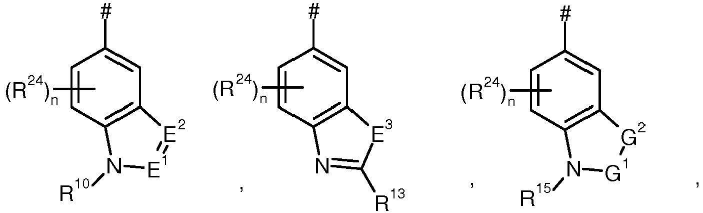 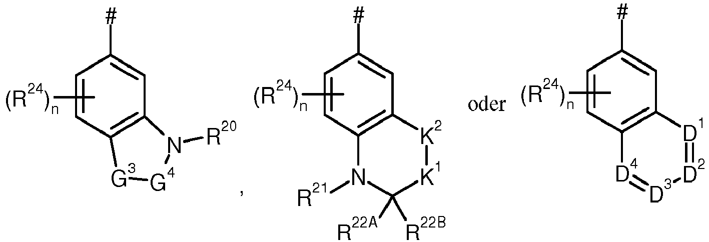 steht, wobei
- #: für die Anknüpfungsstelle an das Uracil-Stickstoffatom steht,
E¹ für CR¹¹ oder N steht,
worin
R¹¹ für Wasserstoff, (C₁-C₄)-Alkyl, (C₃-C₇)-Cycloalkyl oder Aminocarbonyl steht,
E² für CR¹² oder N steht,
worin
R¹² für Wasserstoff, (C₁-C₄)-Alkyl oder (C₃-C₇)-Cycloalkyl steht,
E³ für NR¹⁴ oder S steht,
worin
R¹⁴ für Wasserstoff, (C₁-C₄)-Alkyl oder (C₃-C₇)-Cycloalkyl steht,
G¹ für C=O oder SO₂ steht,
G² für CR^{16A}R^{16B}, NR¹⁷, O oder S steht,
worin
R^{16A} für Wasserstoff, Fluor, (C₁-C₄)-Alkyl oder Hydroxy steht,
R^{16B} für Wasserstoff, Fluor, Chlor, (C₁-C₄)-Alkyl oder Trifluormethyl steht, oder
R^{16A} und R^{16B} bilden zusammen mit dem Kohlenstoffatom, an das sie gebunden sind, einen 3- bis 6-gliedrigen Carbocyclus,
R¹⁷ für Wasserstoff, (C₁-C₆)-Alkyl, (C₃-C₇)-Cycloalkyl oder (C₁-C₄)-Alkoxy-carbonyl steht,
worin (C₁-C₆)-Alkyl mit 1 bis 3 Substituenten unabhängig voneinander ausgewählt aus der Gruppe Fluor, Trifluormethyl, Cyano, (C₃-C₇)-Cycloalkyl, Hydroxy, Trifluormethoxy, (C₁-C₄)-Alkoxy, Azetidinyl, Oxetanyl, Tetrahydrofuranyl und Pyrrolidinyl substituiert sein kann,
G³ für CR^{18A}R^{18B}, NR¹⁹, O oder S steht,
worin
R^{18A} für Wasserstoff, Fluor, (C₁-C₄)-Alkyl oder Hydroxy steht,
R^{18B} für Wasserstoff, Fluor, Chlor, (C₁-C₄)-Alkyl oder Trifluormethyl steht, oder
R^{18A} und R^{18B} zusammen mit dem Kohlenstoffatom, an das sie gebunden sind, einen 3- bis 6-gliedrigen Carbocyclus bilden,
R¹⁹ für Wasserstoff, (C₁-C₆)-Alkyl, (C₃-C₇)-Cycloalkyl oder (C₁-C₄)-Alkoxycarbonyl steht,
worin (C₁-C₆)-Alkyl mit 1 bis 3 Subsituenten unabhängig voneinander ausgewählt aus der Gruppe Fluor, Trifluormethyl, Cyano, (C₃-C₇)-Cycloalkyl, Hydroxy, Trifluormethoxy, (C₁-C₄)-Alkoxy, Azetidinyl, Oxetanyl, Tetrahydrofuranyl und Pyrrolidinyl substituiert sein kann,
G⁴ für CH₂, C=O oder SO₂ steht,
K¹ für CH₂ oder O steht,
K² für CH₂ oder O steht, mit der Massgabe, dass nur eine der Gruppe K¹ und K² für O steht,
D¹, D², D³ und D⁴ unanhängig voneinander für CR²³ oder N stehen,
worin
R²³ für Wasserstoff, Halogen, (C₁-C₆)-Alkyl oder (C₃-C₇)-Cycloalkyl steht, mit der Massgabe, dass maximal 2 der Gruppen D¹, D², D³ und D⁴ für N stehen,
R²⁴ für Fluor oder Methyl steht,
n für eine Zahl 0 oder 1 steht,
R¹⁰ für (C₁-C₄)-Alkyl oder (C₃-C₇)-Cycloalkyl steht,
worin (C₁-C₄)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R¹³ für Wasserstoff, (C₁-C₄)-Alkyl oder (C₃-C₇)-Cycloalkyl steht,
R¹⁵ für Wasserstoff, (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R²⁰ für Wasserstoff, (C₁-C₆)-Alkyl, (C₃-C₇)-Cycloalkyl oder (C₁-C₄)-Alkylcarbonyl steht, worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R²¹ für Wasserstoff, (C₁-C₆)-Alkyl, (C₃-C₇)-Cycloalkyl oder (C₁-C₄)-Alkylsulfonyl steht,
R^{22A} für Wasserstoff oder (C₁-C₄)-Alkyl steht,
R^{22B} für Wasserstoff oder (C₁-C₄)-Alkyl steht, oder
R^{22A} und R^{22B} bilden zusammen mit dem Kohlenstoffatom, an das sie gebunden sind, eine Carbonylgruppe,
sowie ihrer Salze, Solvate und Solvate der Salze zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.

In einer weiteren Ausführungsform der Erfindung sind die Verbindungen geeignet zur Verwendung bei der Behandlung der Endometriose, der Endometriose-assoziierten Fibrose, der Adenomyose und des mit einer Endometrioseerkrankung assoziierten Schmerzes, von Endometriose-assozierten Adhäsionen und Fibrosen, von Endometriose-assoziierten Symptomen wie Dysmenorrhö, Dyspareunie, Dysurie oder Dyschezie sowie von von Endometriose-assoziierter Subfertilität.

In einer weiteren Ausführungsform der Erfindung sind die Verbindungen geeignet zur Verwendung bei der Behandlung und Vorbeugung von postoperativen Adhäsionen in der Bauchhöhle und zur Vorbeugung von adhäsionsbedingter Subfertilität.

In einer weiteren Ausführungsform der Erfindung sind die Verbindungen geeignet zur Behandlung von Morbus Dupuytren (einer Erkrankung des Bindegewebes der Handinnenflächen) und bei der Behandlung und/oder Prophylaxe von chronischen Schmerzen im kleinen Becken.

Bevorzugt im Rahmen der vorliegenden Erfindung ist auch die Verwendung von Verbindungen der Formel (I), in welcher
- R¹: für Wasserstoff, Methyl oder Ethyl steht,
- R²: für eine Gruppe der Formel 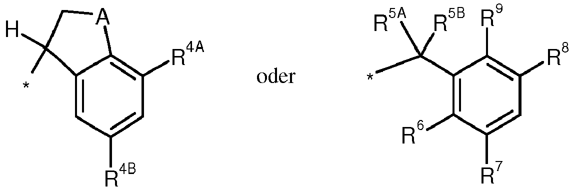 steht, wobei
* für die Anknüpfungsstelle an das Uracil-Stickstoffatom steht,
A für -CH₂-, -CH₂-CH₂-, -O-CH₂-## oder Sauerstoff steht,
worin
## für die Anknüpfstelle an den Phenyl-Ring steht,
R^{4A} für Wasserstoff, Fluor, Chlor, Trifluormethyl oder Methyl steht,
R^{4B} für Wasserstoff, Fluor, Chlor, Trifluormethyl oder Methyl steht,
mit der Massgabe, dass mindestens einer der Reste R^{4A} und R^{4B} von Wasserstoff verschieden ist,
- R^{5A}: für Wasserstoff steht,
- R^{5B}: für Wasserstoff steht,
- R⁶: für Wasserstoff steht,
- R⁷: für Wasserstoff steht,
- R⁸: für Fluor, Chlor, Difluormethyl, Trifluormethyl oder Methyl steht,
R⁹ für Fluor, Chlor, Difluormethyl, Trifluormethyl oder Methyl steht,
- R³: für eine Gruppe der Formel 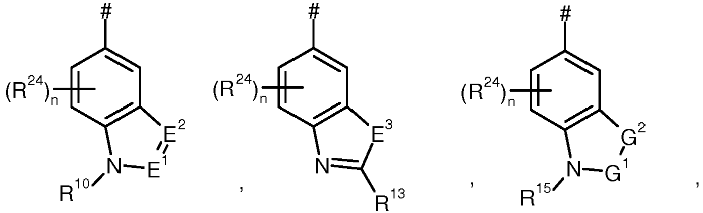 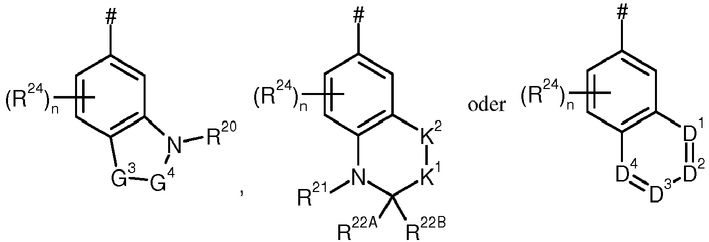 steht, wobei
# für die Anknüpfungsstelle an das Uracil-Stickstoffatom steht,
E¹ für CR¹¹ oder N steht,
worin
R¹¹ für Wasserstoff, (C₁-C₄)-Alkyl, (C₃-C₇)-Cycloalkyl oder Aminocarbonyl steht,
E² für CR¹² oder N steht,
worin
R¹² für Wasserstoff, (C₁-C₄)-Alkyl oder (C₃-C₇)-Cycloalkyl steht,
E³ für NR¹⁴ oder S steht,
worin
R¹⁴ für Wasserstoff, (C₁-C₄)-Alkyl oder (C₃-C₇)-Cycloalkyl steht,
G¹ für C=O oder SO₂ steht,
G² für CR^{16A}R^{16B}, NR¹⁷, O oder S steht,
worin
R^{16A} für Wasserstoff, Fluor, (C₁-C₄)-Alkyl oder Hydroxy steht,
R^{16B} für Wasserstoff, Fluor, Chlor, (C₁-C₄)-Alkyl oder Trifluormethyl steht, oder
R^{16A} und R^{16B} bilden zusammen mit dem Kohlenstoffatom, an das sie gebunden sind, einen 3- bis 6-gliedrigen Carbocyclus,
R¹⁷ für Wasserstoff, (C₁-C₆)-Alkyl, (C₃-C₇)-Cycloalkyl oder (C₁-C₄)-Alkoxycarbonyl steht,
worin (C₁-C₆)-Alkyl mit 1 bis 3 Substituenten unabhängig voneinander ausgewählt aus der Gruppe Fluor, Trifluormethyl, Cyano, (C₃-C₇)-Cycloalkyl, Hydroxy, Trifluormethoxy, (C₁-C₄)-Alkoxy, Azetidinyl, Oxetanyl, Tetrahydrofuranyl und Pyrrolidinyl substituiert sein kann,
G³ für CR^{18A}R^{18B}, NR¹⁹, O oder S steht,
worin
R^{18A} für Wasserstoff, Fluor, (C₁-C₄)-Alkyl oder Hydroxy steht,
R^{18B} für Wasserstoff, Fluor, Chlor, (C₁-C₄)-Alkyl oder Trifluormethyl steht, oder
R^{18A} und R^{18B} zusammen mit dem Kohlenstoffatom, an das sie gebunden sind, einen 3- bis 6-gliedrigen Carbocyclus bilden,
R¹⁹ für Wasserstoff, (C₁-C₆)-Alkyl, (C₃-C₇)-Cycloalkyl oder (C₁-C₄)-Alkoxycarbonyl steht,
worin (C₁-C₆)-Alkyl mit 1 bis 3 Subsituenten unabhängig voneinander ausgewählt aus der Gruppe Fluor, Trifluormethyl, Cyano, (C₃-C₇)-Cycloalkyl, Hydroxy, Trifluormethoxy, (C₁-C₄)-Alkoxy, Azetidinyl, Oxetanyl, Tetrahydrofuranyl und Pyrrolidinyl substituiert sein kann,
G⁴ für CH₂, C=O oder SO₂ steht,
K¹ für CH₂ oder O steht,
K² für CH₂ oder O steht, mit der Massgabe, dass nur eine der Gruppe K¹ und K² für O steht,
D¹, D², D³ und D⁴ unanhängig voneinander für CR²³ oder N stehen,
worin
R²³ für Wasserstoff, Halogen, (C₁-C₆)-Alkyl oder (C₃-C₇)-Cycloalkyl steht,
mit der Massgabe, dass maximal 2 der Gruppen D¹, D², D³ und D⁴ für N stehen,
- R²⁴: für Fluor oder Methyl steht,
- n: für eine Zahl 0 oder 1 steht,
- R¹⁰: für (C₁-C₄)-Alkyl oder (C₃-C₇)-Cycloalkyl steht,
worin (C₁-C₄)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
- R¹³: für Wasserstoff, (C₁-C₄)-Alkyl oder (C₃-C₇)-Cycloalkyl steht,
- R¹⁵: für Wasserstoff, (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
- R²⁰: für Wasserstoff, (C₁-C₆)-Alkyl, (C₃-C₇)-Cycloalkyl oder (C₁-C₄)-Alkylcarbonyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
- R²¹: für Wasserstoff, (C₁-C₆)-Alkyl, (C₃-C₇)-Cycloalkyl oder (C₁-C₄)-Alkylsulfonyl steht,
- R^{22A}: für Wasserstoff oder (C₁-C₄)-Alkyl steht,
- R^{22B}: für Wasserstoff oder (C₁-C₄)-Alkyl steht,
oder
- R^{22A} und R^{22B}: bilden zusammen mit dem Kohlenstoffatom, an das sie gebunden sind, eine Carbonylgruppe
sowie ihrer Salze, Solvate und Solvate der Salze zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.

In einer weiteren Ausführungsform der Erfindung sind die Verbindungen geeignet zur Verwendung bei der Behandlung der Endometriose, der Endometriose-assoziierten Fibrose, der Adenomyose und des mit einer Endometrioseerkrankung assoziierten Schmerzes, von Endometriose-assozierten Adhäsionen und Fibrosen, von Endometriose-assoziierten Symptomen wie Dysmenorrhö, Dyspareunie, Dysurie oder Dyschezie sowie von von Endometriose-assoziierter Subfertilität.

In einer weiteren Ausführungsform der Erfindung sind die Verbindungen geeignet zur Verwendung bei der Behandlung und Vorbeugung von postoperativen Adhäsionen in der Bauchhöhle und zur Vorbeugung von adhäsionsbedingter Subfertilität.

In einer weiteren Ausführungsform der Erfindung sind die Verbindungen geeignet zur Behandlung von Morbus Dupuytren (einer Erkrankung des Bindegewebes der Handinnenflächen) und bei der Behandlung und/oder Prophylaxe von chronischen Schmerzen im kleinen Becken.

Besonders bevorzugt im Rahmen der vorliegenden Erfindung ist die Verwendung von Verbindungen der Formel (I), in welcher
- R¹: für Wasserstoff steht,
- R²: für eine Gruppe der Formel 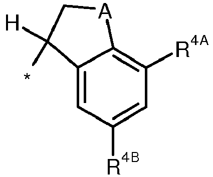 steht, wobei
* für die Anknüpfungsstelle an das Uracil-Stickstoffatom steht,
A für-CH₂-steht,
R^{4A} für Chlor oder Trifluormethyl steht,
R^{4B} für Wasserstoff steht,
- R³: für eine Gruppe der Formel 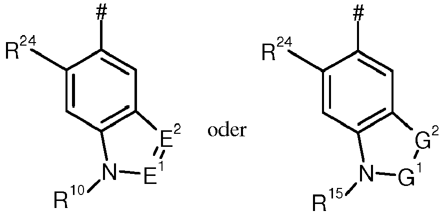 steht, wobei
# für die Anknüpfungsstelle an das Uracil-Stickstoffatom steht,
E¹ für CR¹¹ steht,
worin
R¹¹ für Wasserstoff steht,
E² für N steht,
G¹ für C=O steht,
G² für CR^{16A}R^{16B}, NR¹⁷, O oder S steht,
worin
R^{16A} für Wasserstoff, Fluor, Methyl oder Hydroxy steht,
R^{16B} für Wasserstoff, Fluor, Methyl oder Trifluormethyl steht, oder
R^{16A} und R^{16B} zusammen mit dem Kohlenstoffatom, an das sie gebunden sind, einen Cyclopropyl-Ring bilden,
R¹⁷ für Wasserstoff, (C₁-C₄)-Alkyl oder (C₃-C₅)-Cycloalkyl steht,
worin (C₁-C₄)-Alkyl mit 1 bis 3 Substituenten unabhängig voneinander ausgewählt aus der Gruppe Fluor, Trifluormethyl, Cyano, Cyclopropyl, Cyclobutyl, Hydroxy, Trifluormethoxy, Methoxy, Ethoxy, Azetidinyl, Oxetanyl, Tetrahydrofuranyl und Pyrrolidinyl substituiert sein kann,
R²⁴ für Wasserstoff oder Fluor steht,
R¹⁰ für (C₁-C₄)-Alkyl steht,
R¹⁵ für Wasserstoff, Methyl oder Ethyl steht,
worin Methyl und Ethyl mit 1 Substituenten ausgewählt aus der Gruppe Fluor, Trifluormethyl und Cyclopropyl substituiert sein können,
sowie ihrer Salze, Solvate und Solvate der Salze zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.

In einer weiteren Ausführungsform der Erfindung sind die Verbindungen geeignet zur Verwendung bei der Behandlung der Endometriose, der Endometriose-assoziierten Fibrose, der Adenomyose und des mit einer Endometrioseerkrankung assoziierten Schmerzes, von Endometriose-assozierten Adhäsionen und Fibrosen, von Endometriose-assoziierten Symptomen wie Dysmenorrhö, Dyspareunie, Dysurie oder Dyschezie sowie von von Endometriose-assoziierter Subfertilität.

In einer weiteren Ausführungsform der Erfindung sind die Verbindungen geeignet zur Verwendung bei der Behandlung und Vorbeugung von postoperativen Adhäsionen in der Bauchhöhle und zur Vorbeugung von adhäsionsbedingter Subfertilität.

In einer weiteren Ausführungsform der Erfindung sind die Verbindungen geeignet zur Behandlung von Morbus Dupuytren (einer Erkrankung des Bindegewebes der Handinnenflächen) und bei der Behandlung und/oder Prophylaxe von chronischen Schmerzen im kleinen Becken.

Bevorzugt ist im Rahmen der vorliegenden Erfindung auch die Verwendung von Verbindungen der Formel (I), in welcher
- R¹: für Wasserstoff steht,
- R²: für eine Gruppe der Formel 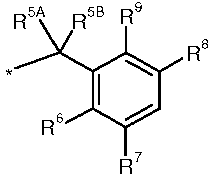 steht, wobei
* für die Anknüpfungsstelle an das Uracil-Stickstoffatom steht,
R^{5A} für Wasserstoff steht,
R^{5B} für Wasserstoff steht,
R⁶ für Wasserstoff steht,
R⁷ für Wasserstoff steht,
R⁸ für Fluor, Chlor oder Trifluormethyl steht,
R⁹ für Fluor, Chlor, Trifluormethyl oder Methyl steht,
- R³: für eine Gruppe der Formel 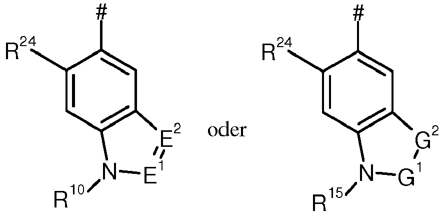 steht, wobei
# für die Anknüpfungsstelle an das Uracil-Stickstoffatom steht,
E¹ für CR¹¹ steht,
worin
R¹¹ für Wasserstoff steht,
E² für N steht,
G¹ für C=O steht,
G² für CR^{16A}R^{16B}, NR¹⁷, O oder S steht,
worin
R^{16A} für Wasserstoff, Fluor, Methyl oder Hydroxy steht,
R^{16B} für Wasserstoff, Fluor, Methyl oder Trifluormethyl steht, oder
R^{16A} und R^{16B} zusammen mit dem Kohlenstoffatom, an das sie gebunden sind, einen Cyclopropyl-Ring bilden,
R¹⁷ für Wasserstoff, (C₁-C₄)-Alkyl oder (C₃-C₅)-Cycloalkyl steht,
worin (C₁-C₄)-Alkyl mit 1 bis 3 Substituenten unabhängig voneinander ausgewählt aus der Gruppe Fluor, Trifluormethyl, Cyano, Cyclopropyl, Cyclobutyl, Hydroxy, Trifluormethoxy, Methoxy, Ethoxy, Azetidinyl, Oxetanyl, Tetrahydrofuranyl und Pyrrolidinyl substituiert sein kann,
R²⁴ für Wasserstoff oder Fluor steht,
R¹⁰ für (C₁-C₄)-Alkyl steht,
R¹⁵ für Wasserstoff, Methyl oder Ethyl steht,
worin Methyl und Ethyl mit 1 Substituenten ausgewählt aus der Gruppe Fluor, Trifluormethyl und Cyclopropyl substituiert sein können,
sowie ihrer Salze, Solvate und Solvate der Salze zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.

In einer weiteren Ausführungsform der Erfindung sind die Verbindungen geeignet zur Verwendung bei der Behandlung der Endometriose, der Endometriose-assoziierten Fibrose, der Adenomyose und des mit einer Endometrioseerkrankung assoziierten Schmerzes, von Endometriose-assozierten Adhäsionen und Fibrosen, von Endometriose-assoziierten Symptomen wie Dysmenorrhö, Dyspareunie, Dysurie oder Dyschezie sowie von von Endometriose-assoziierter Subfertilität.

In einer weiteren Ausführungsform der Erfindung sind die Verbindungen geeignet zur Verwendung bei der Behandlung und Vorbeugung von postoperativen Adhäsionen in der Bauchhöhle und zur Vorbeugung von adhäsionsbedingter Subfertilität.

In einer weiteren Ausführungsform der Erfindung sind die Verbindungen geeignet zur Behandlung von Morbus Dupuytren (einer Erkrankung des Bindegewebes der Handinnenflächen) und bei der Behandlung und/oder Prophylaxe von chronischen Schmerzen im kleinen Becken.

Bevorzugt ist im Rahmen der vorliegenden Erfindung auch die Verwendung von Verbindungen der Formel (I), in welcher
- R¹: für Wasserstoff, Methyl oder Ethyl steht,
- R²: für eine Gruppe der Formel 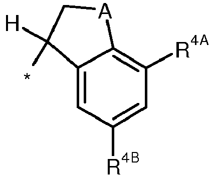 steht, wobei
* für die Anknüpfungsstelle an das Uracil-Stickstoffatom steht,
A für-CH₂-steht,
R^{4A} für Chlor oder Trifluormethyl steht,
R^{4B} für Wasserstoff steht,
sowie ihrer Salze, Solvate und Solvate der Salze zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.

In einer weiteren Ausführungsform der Erfindung sind die Verbindungen geeignet zur Verwendung bei der Behandlung der Endometriose, der Endometriose-assoziierten Fibrose, der Adenomyose und des mit einer Endometrioseerkrankung assoziierten Schmerzes, von Endometriose-assozierten Adhäsionen und Fibrosen, von Endometriose-assoziierten Symptomen wie Dysmenorrhö, Dyspareunie, Dysurie oder Dyschezie sowie von von Endometriose-assoziierter Subfertilität.

In einer weiteren Ausführungsform der Erfindung sind die Verbindungen geeignet zur Verwendung bei der Behandlung und Vorbeugung von postoperativen Adhäsionen in der Bauchhöhle und zur Vorbeugung von adhäsionsbedingter Subfertilität.

In einer weiteren Ausführungsform der Erfindung sind die Verbindungen geeignet zur Behandlung von Morbus Dupuytren (einer Erkrankung des Bindegewebes der Handinnenflächen) und bei der Behandlung und/oder Prophylaxe von chronischen Schmerzen im kleinen Becken.

Bevorzugt ist im Rahmen der vorliegenden Erfindung auch die Verwendung von Verbindungen der Formel (I), in welcher
- R³: für eine Gruppe der Formel 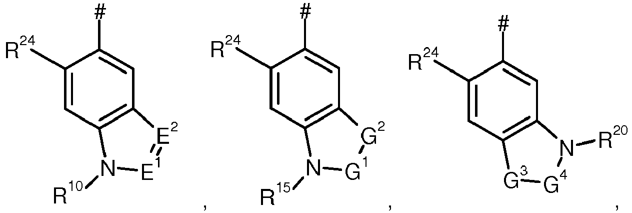 oder 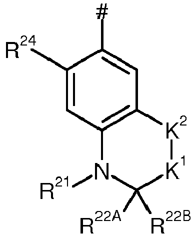 steht, wobei
# für die Anknüpfungsstelle an das Uracil-Stickstoffatom steht,
E¹ für CR¹¹ oder N steht,
worin
R¹¹ für Wasserstoff, Methyl, Ethyl oder Aminocarbonyl steht,
E² für CR¹² oder N steht,
worin
R¹² für Wasserstoff steht,
G¹ für C=O oder SO₂ steht,
G² für CR^{16A}R^{16B}, NR¹⁷, O oder S steht,
worin
R^{16A} für Wasserstoff, Fluor, Methyl oder Hydroxy steht,
R^{16B} für Wasserstoff, Fluor, Chlor, Methyl oder Trifluormethyl steht, oder
R^{16A} und R^{16B} bilden zusammen mit dem Kohlenstoffatom, an das sie gebunden sind, einen Cyclopropyl-Ring,
R¹⁷ für Wasserstoff, (C₁-C₄)-Alkyl, Cyclopropyl oder Cyclobutyl steht,
worin (C₁-C₄)-Alkyl mit 1 oder 2 Substituenten unabhängig voneinander ausgewählt aus der Gruppe Fluor, Trifluormethyl, Cyclopropyl, Cyclobutyl, Hydroxy, Azetidinyl und Oxetanyl substituiert sein kann,
G³ für CR^{18A}R^{18B} steht,
worin
R^{18A} für Wasserstoff, Fluor, Methyl oder Hydroxy steht,
R^{18B} für Wasserstoff, Fluor, Methyl oder Trifluormethyl steht,
G⁴ für C=O steht,
K¹ für CH₂ oder O steht,
K² für CH₂ steht,
R²⁴ für Wasserstoff, Fluor oder Methyl steht,
R¹⁰ für Methyl oder Ethyl steht,
R¹⁵ für Methyl oder Ethyl steht,
R²⁰ für Wasserstoff, Methyl, Ethyl oder Methylcarbonyl steht,
R²¹ für Methyl oder Ethyl steht,
R^{22A} und R^{22B} bilden zusammen mit dem Kohlenstoffatom, an das sie gebunden sind, eine Carbonylgruppe,
sowie ihrer Salze, Solvate und Solvate der Salze zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.

In einer weiteren Ausführungsform der Erfindung sind die Verbindungen geeignet zur Verwendung bei der Behandlung der Endometriose, der Endometriose-assoziierten Fibrose, der Adenomyose und des mit einer Endometrioseerkrankung assoziierten Schmerzes, von Endometriose-assozierten Adhäsionen und Fibrosen, von Endometriose-assoziierten Symptomen wie Dysmenorrhö, Dyspareunie, Dysurie oder Dyschezie sowie von von Endometriose-assoziierter Subfertilität.

In einer weiteren Ausführungsform der Erfindung sind die Verbindungen geeignet zur Verwendung bei der Behandlung und Vorbeugung von postoperativen Adhäsionen in der Bauchhöhle und zur Vorbeugung von adhäsionsbedingter Subfertilität.

In einer weiteren Ausführungsform der Erfindung sind die Verbindungen geeignet zur Behandlung von Morbus Dupuytren (einer Erkrankung des Bindegewebes der Handinnenflächen) und bei der Behandlung und/oder Prophylaxe von chronischen Schmerzen im kleinen Becken.

Bevorzugt ist im Rahmen der vorliegenden Erfindung auch die Verwendung von Verbindungen der Formel (I), in welcher
- R²: für eine Gruppe der Formel 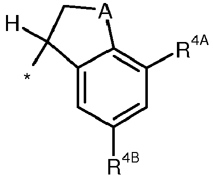 steht, wobei
* für die Anknüpfungsstelle an das Uracil-Stickstoffatom steht,
A für-CH₂-steht,
R^{4A} für Chlor oder Trifluormethyl steht,
R^{4B} für Wasserstoff steht,
und das Kohlenstoffatom, das an das Uracilstickstoffatom gebunden ist, eine R-Konfiguration hat,
sowie ihrer Salze, Solvate und Solvate der Salze zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.

In einer weiteren Ausführungsform der Erfindung sind die Verbindungen geeignet zur Verwendung bei der Behandlung der Endometriose, der Endometriose-assoziierten Fibrose, der Adenomyose und des mit einer Endometrioseerkrankung assoziierten Schmerzes, von Endometriose-assozierten Adhäsionen und Fibrosen, von Endometriose-assoziierten Symptomen wie Dysmenorrhö, Dyspareunie, Dysurie oder Dyschezie sowie von von Endometriose-assoziierter Subfertilität.

In einer weiteren Ausführungsform der Erfindung sind die Verbindungen geeignet zur Verwendung bei der Behandlung und Vorbeugung von postoperativen Adhäsionen in der Bauchhöhle und zur Vorbeugung von adhäsionsbedingter Subfertilität.

In einer weiteren Ausführungsform der Erfindung sind die Verbindungen geeignet zur Behandlung von Morbus Dupuytren (einer Erkrankung des Bindegewebes der Handinnenflächen) und bei der Behandlung und/oder Prophylaxe von chronischen Schmerzen im kleinen Becken.

Bevorzugt ist im Rahmen der vorliegenden Erfindung auch die Verwendung von Verbindungen der Formel (I), in welcher
- R²: für eine Gruppe der Formel 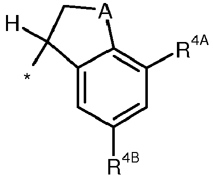 steht, wobei
* für die Anknüpfungsstelle an das Uracil-Stickstoffatom steht,
A für-CH₂-steht,
R^{4A} für Wasserstoff, Fluor, Chlor, Trifluormethyl oder Methyl steht,
R^{4B} für Wasserstoff, Fluor, Chlor, Trifluormethyl oder Methyl steht,
mit der Maßgabe, dass mindestens einer der Reste R^{4A} und R^{4B} von Wasserstoff verschieden ist,
und das Kohlenstoffatom, das an das Uracilstickstoffatom gebunden ist, eine R-Konfiguration hat,
sowie ihrer Salze, Solvate und Solvate der Salze zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.

In einer weiteren Ausführungsform der Erfindung sind die Verbindungen geeignet zur Verwendung bei der Behandlung der Endometriose, der Endometriose-assoziierten Fibrose, der Adenomyose und des mit einer Endometrioseerkrankung assoziierten Schmerzes, von Endometriose-assozierten Adhäsionen und Fibrosen, von Endometriose-assoziierten Symptomen wie Dysmenorrhö, Dyspareunie, Dysurie oder Dyschezie sowie von von Endometriose-assoziierter Subfertilität.

In einer weiteren Ausführungsform der Erfindung sind die Verbindungen geeignet zur Verwendung bei der Behandlung und Vorbeugung von postoperativen Adhäsionen in der Bauchhöhle und zur Vorbeugung von adhäsionsbedingter Subfertilität.

In einer weiteren Ausführungsform der Erfindung sind die Verbindungen geeignet zur Behandlung von Morbus Dupuytren (einer Erkrankung des Bindegewebes der Handinnenflächen) und bei der Behandlung und/oder Prophylaxe von chronischen Schmerzen im kleinen Becken.

Die Verbindungen der allgemeinen Formel (I) können allein oder bei Bedarf in Kombination mit anderen Wirkstoffen eingesetzt werden.

Weiterer Gegenstand der Erfindung ist daher auch eine Verwendung der Chymaseinhibitoren der allgemeinen Formel (I) sowie ihrer Salze, Solvate und Solvate der Salze in Kombination mit anderen Wirkstoffen zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.
In einer weiteren Ausführungsform der Erfindung sind die Verbindungen geeignet zur Verwendung bei der Behandlung der Endometriose, der Endometriose-assoziierten Fibrose, der Adenomyose und des mit einer Endometrioseerkrankung assoziierten Schmerzes, von Endometriose-assozierten Adhäsionen und Fibrosen, von Endometriose-assoziierten Symptomen wie Dysmenorrhö, Dyspareunie, Dysurie oder Dyschezie sowie von von Endometriose-assoziierter Subfertilität.

In einer weiteren Ausführungsform der Erfindung sind die Verbindungen der allgemeinen Formel (I) sowie ihrer Salze, Solvate und Solvate der Salze in Kombination mit anderen Wirkstoffen geeignet zur Verwendung bei der Behandlung und Vorbeugung von postoperativen Adhäsionen in der Bauchhöhle und zur Vorbeugung von adhäsionsbedingter Subfertilität.

In einer weiteren Ausführungsform der Erfindung sind die Verbindungen der allgemeinen Formel (I) sowie ihrer Salze, Solvate und Solvate der Salze in Kombination mit anderen Wirkstoffen geeignet zur Behandlung von Morbus Dupuytren (einer Erkrankung des Bindegewebes der Handinnenflächen) und bei der Behandlung und/oder Prophylaxe von chronischen Schmerzen im kleinen Becken.

Geeignete Wirkstoffe, die in Kombination mit den Chymaseinhibitoren gemäß allgemeiner Formel (I) verwendet werden können, werden im Folgenden beispielhaft und nicht limitierend genannt: bekannte hormonelle Agentien wie Progestine, z. B. Norethynodrel, Norethindrone, Norethindronacetat, Ethynodiolacetat, Norgestrel, Levonorgestrel, Norgestimat, Desogestrel, Gestoden, Drospirenon, Dienogest oder Nomegestrolacetat, allein oder zusammen mit Estrogenen, z. B. Ethinylestradiol, Estradiol oder Estradiolester wie beispielsweise Estradiolvalerat, Dabei ist auch die Kombination mit hormonellen Kontrazeptiva möglich, die entweder oral, subkutan, transdermal, intrauterin oder intravaginal verabreicht werden können.

Geeignete Präparate sind hierfür Kombinierte Orale Kontrazeptiva (COC), Progestin-Only-Pille (POP), welche nur ein Progestin enthält oder Hormon-enthaltende Vorrichtungen wie Implantate, Pflaster oder intravaginale Ringe.
Beispiele für COCs beinhalten eine Kombination eines Estrogens (Estradiol) und eines Gestagens (Progestin). Die estrogene Komponente in den meisten COCs ist Ethinylestradiol. Einige COCs enthalten Estradiol oder Estradiolvalerat. Folgende Progestine werden in COCs verwendet: Norethynodrel, Norethindron, Norethindronacetat, Ethynodiolacetat, Norgestrel, Levonorgestrel, Norgestimat, Desogestrel, Gestoden, Drospirenon, Dienogest oder Nomegestrolacetat.

Eine weitere Ausführungsform der vorliegenden Erfindung ist daher eine Kombination von COC mit den vorliegenden Verbindungen der allgemeinen Formel (I), beispielsweise Kombinationen der Verbindungen der allgemeinen Formel (I) mit Ethinylestradiol und Drospirenon (Yasmin^{®} und Yaz^{®}), mit Ethinylestradiol und Levonorgestrel (Microgynon^{®} und Miranova^{®}), mit Ethinylestradiol und Desogestrel (Marvelon^{®}), mit Ethinylestradiol und Dienogest (Valette^{®}) oder Ethinylestradiol mit Chlormadinonacetat (Belara^{®} und Enriqa^{®}). Eine weitere Ausführungsform der vorliegenden Erfindung ist eine Kombination von Verbindungen der allgemeinen Formel (I) mit beispielsweise Estradiol und Normegestrol (Zoely^{®}) oder Estradiolvalerat und Dienogest (Qlaira^{®}).

Eine weitere Ausführungsform ist die Verabreichung von Verbindungen der allgemeinen Formel (I) in Kombination mit synthetischen Progestinen ohne Estrogenkomponente. Diese Variante lässt sich beispielsweise mit sogenannten POPs (**P**rogestin-**O**nly-**P**ills) als Kontrazeptiva realisieren. POPs sind auch als Minipillen bekannt. Beispiele für POPs sind Cerazette^{®} mit dem Progestin Desogestrel, Microlut^{®} mit Levonorgestrel oder Micronor^{®} mit Norethindron.

Eine weitere Ausführungsform der vorliegenden Erfindung beinhaltet eine Kombination der Verbindungen der allgemeinen Formel (I) mit nicht-oralen Progestin-Only Formen wie intrauterine Systeme (IUDs), z.B. Mirena^{®}, Jaydess^{®} oder Kyleena^{®} jeweils mit Levonorgestrel, oder wie injizierbare Formen, z.B. Depo-Provera^{®} mit Medroxyprogesteronacetat, oder wie Implantate, z.B. Implanon mit Etonogestrel.

Weitere Hormone-enthaltende Formen mit kontrazeptivem Effekt, die mit den Verbindungen der allgemeinen Formel (I) kombiniert werden können, sind Vaginalringe wie der NuvaRing^{®} mit Ethinylestradiol und Etonogestrel oder Vaginalringe, die einen Aromatasehemmer, beispielsweise Anastrozol, und ein Gestagen enthalten, oder transdermale Systeme wie kontrazeptive Pflaster, z.B. Ortho-Evra mit Ethinylestradiol und Norelgestromin oder Apleek (Lisvy) mit Ethinylestradiol und Gestoden.

Weiterhin können die Verbindungen der allgemeinen Formel (I) in Kombination mit folgenden Wirkstoffen im Sinne der vorliegenden Erfindung verwendet werden: Kombination mit Inhibitoren der P2X-Purinrezeptoren (P2X3, P2X4), mit Inhibitoren von IRAK4 (Interleukin-1 Rezeptor-Associated Kinase 4), PTGES (Prostaglandin-E-synthase) und Antagonisten des Prostaglandinrezeptors EP4 (Prostaglandin E2 Rezeptor 4). Weitere Kombinationen sind mit AKR1C3-Inhibitoren (Aldoketoreduktase 1 C3) und mit funktionsblockierenden Antikörpern des Prolaktinrezeptors.

Die genannten Kombinationen sind geeignet zur Verwendung bei der Behandlung von entzündlichen und unterschiedlichen fibrotischen Erkrankungen, insbesondere zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.

In einer weiteren Ausführungsform der Erfindung sind die genannten Kombinationen geeignet zur Verwendung bei der Behandlung der Endometriose, der Endometrioseassoziierten Fibrose, der Adenomyose und des mit einer Endometrioseerkrankung assoziierten Schmerzes, von Endometriose-assozierten Adhäsionen und Fibrosen, von Endometriose-assoziierten Symptomen wie Dysmenorrhö, Dyspareunie, Dysurie oder Dyschezie sowie von von Endometriose-assoziierter Subfertilität.

In einer weiteren Ausführungsform der Erfindung sind die genannten Kombinationen geeignet zur Verwendung bei der Behandlung und Vorbeugung von postoperativen Adhäsionen in der Bauchhöhle und zur Vorbeugung von adhäsionsbedingter Subfertilität.

In einer weiteren Ausführungsform der Erfindung sind die genannten Kombinationen geeignet zur Behandlung von Morbus Dupuytren (einer Erkrankung des Bindegewebes der Handinnenflächen) und bei der Behandlung und/oder Prophylaxe von chronischen Schmerzen im kleinen Becken.

Die Verbindungen der allgemeinen Formel (I) können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.
Für diese Applikationswege können die Verbindungen der allgemeinen Formel (I) in geeigneten Applikationsformen verabreicht werden.
Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.
Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.
Für die sonstigen Applikationswege eignen sich z.B. Inhalationsarzneiformen (u.a. Pulverinhalatoren, Nebulizer, Aerosole), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.
Bevorzugt sind die orale oder parenterale Applikation, insbesondere die orale, die intravenöse und die inhalative Applikation.
Die Verbindungen der allgemeinen Formel (I) können in die angeführten Applikationsformen überführt werden. Dies kann in an sich bekannter Weise durch Mischen mit inerten, nicht-toxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.
Im Allgemeinen hat es sich als vorteilhaft erwiesen, bei oraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.
Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.
Die nachfolgenden Ausführungsbeispiele erläutern die Erfindung. Die Erfindung ist nicht auf die Beispiele beschränkt.
Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich, sofern nicht anders angegeben, jeweils auf das Volumen.

### Experimenteller Teil

Die Synthese der Verbindungen der allgemeinen Formel (I) ist in WO2013/167495 A1 beschrieben.

### Bewertung der pharmakologischen Wirksamkeit

Die pharmakologische Wirkung der erfindungsgemäßen Verbindungen auf Chymase wird in WO2013/167495 A1 mit den dort beschriebenen Assays gezeigt.

Der Vollständigkeit halber sind die Ergebnisse des enzymatischen Chymase-Assays hier noch einmal aufgeführt.

### Enzymatischer Chymase-Assay (WO2013/167495)

Als Enzymquelle wird rekombinante humane Chymase (exprimiert in HEK293 Zellen) oder Chymase aufgereinigt aus Hamsterzungen benutzt. Als Substrat für Chymase wird Abz-HPFHL-Lys(Dnp)-NH¬2 benutzt. Für den Assay werden 1 µl einer 50-fach konzentrierten Lösung von Testsubstanz in DMSO, 24 µl Enzymlösung (Verdünnung 1:80.000 human oder 1:4.000 Hamster) und 25 µl Substratlösung (finale Konzentration 10 µM) in Assaypuffer (Tris 50 mM (pH 7.5), Natriumchlorid 150 mM, BSA 0.10 %, Chaps 0.10 %, Glutathion 1 mM, EDTA 1 mM) in einer weißen 384-Loch Mikrotiterplatte (Greiner Bio-One, Frickenhausen, Deutschland) zusammengegeben. Die Reaktion wird 60 min bei 32 Grad inkubiert und die Fluoreszenz-Emission bei 465 nm nach Anregung mit 340 nm wird in einem Fluoreszenz-Reader z.B. Tecan Ultra (Tecan, Männedorf, Schweiz) gemessen.

Eine Testverbindung wird auf der gleichen Mikrotiterplatte in 10 verschiedenen Konzentrationen von 30 µM bis 1 nM in Doppelbestimmung getestet. Die Daten werden normalisiert (Enzymreaktion ohne Inhibitor = 0 % Inhibition, alle Assaykomponenten ohne Enzym = 100 % Inhibition) und IC50-Werte mit einer hauseigenen Software kalkuliert. Verbindungen im Sinne der Erfindung, die in diesem Assay getestet wurden, hemmten die Chymaseaktivität mit einem IC50-Wert kleiner 10 µM.

Für die erfindungsgemäßen Verbindungen repräsentative IC50-Werte sind in den folgenden Tabellen 1 und 2 wiedergegeben:

**Tabelle 1:**

| **Beispiel Nr.** | **Hamster Chymase IC 50 [nM]** | **Beispiel Nr.** | **Hamster Chymase IC 50 [nM]** | **Beispiel Nr.** | **Hamster Chymase IC 50 [nM]** |
|---|---|---|---|---|---|
| 1 | 8 | 117 | 3 | 191 | 16 |
| 2 | 7 | 118 | 6 | 192 | 5 |
| 3 | 9 | 119 | 280 | 193 | 8 |
| 4 | 64 | 120 | 1025 | 194 | 13 |
| 5 | 20 | 121 | 3 | 195 | 4 |
| 8 | 33 | 122 | 2 | 196 | 6 |
| 9 | 1500 | 123 | 4 | 197 | 10 |
| 10 | 1600 | 124 | 7 | 198 | 54 |
| 13 | 5 | 125 | 6 | 199 | 8 |
| 14 | 10 | 126 | 10 | 200 | 4 |
| 15 | 330 | 127 | 34 | 201 | 7 |
| 16 | 14 | 128 | 7 | 202 | 4 |
| 18 | 10 | 129 | 450 | 203 | 20 |
| 20 | 8 | 130 | 350 | 204 | 39 |
| 21 | 5 | 131 | 4 | 205 | 3 |
| 22 | 6 | 132 | 2 | 206 | 3 |
| 25 | 7 | 133 | 465 | 207 | 4 |
| 27 | 5 | 134 | 2 | 209 | 13 |
| 28 | 4 | 135 | 4 | 211 | 20 |
| 33 | 4 | 136 | 2 | 213 | 18 |
| 34 | 7 | 137 | 4 | 214 | 20 |
| 35 | 6 | 138 | 4 | 215 | 26 |
| 37 | 700 | 139 | 2 | 216 | 183 |
| 40 | 15 | 140 | 1 | 217 | 1 |
| 41 | 23 | 141 | 2 | 218 | 4 |
| 42 | 7 | 142 | 1 | 219 | 5 |
| 43 | 643 | 143 | 2 | 220 | 6 |
| 44 | 18 | 144 | 2 | 221 | 10 |
| 45 | 50 | 145 | 2 | 222 | 12 |
| 47 | 35 | 146 | 1 | 223 | 3 |
| 48 | 17 | 147 | 2 | 224 | 2 |
| 49 | 17 | 148 | 4 | 225 | 4 |
| 50 | 31 | 149 | 2 | 226 | 3 |
| 51 | 120 | 150 | 5 | 227 | 2 |
| 52 | 16 | 151 | 2 | 228 | 14 |
| 53 | 30 | 152 | 19 | 229 | 4 |
| 55 | 39 | 153 | 4 | 230 | 170 |
| 56 | 67 | 154 | 4 | 231 | 21 |
| 62 | 44 | 155 | 5 | 232 | 6 |
| 63 | 37 | 156 | 12 | 233 | 470 |
| 64 | 19 | 157 | 6 | 234 | 270 |
| 65 | 19 | 158 | 10 | 235 | 9 |
| 66 | 30 | 159 | 92 | 236 | 5 |
| 67 | 4 | 160 | 32 | 238 | 45 |
| 75 | 82 | 161 | 53 | 239 | 490 |
| 76 | 41 | 162 | 58 | 240 | 67 |
| 77 | 170 | 163 | 28 | 241 | 2 |
| 78 | 140 | 164 | 34 | 242 | 40 |
| 79 | 210 | 165 | 40 | 243 | 6 |
| 81 | 65 | 166 | 62 | 244 | 2 |
| 82 | | 167 | 91 | 245 | 67 |
| 83 | 220 | 168 | 49 | 246 | 1 |
| 86 | 140 | 169 | 370 | 247 | 1 |
| 89 | 84 | 170 | 20 | 248 | 2 |
| 94 | 62 | 171 | 17 | 249 | 200 |
| 95 | 100 | 172 | 27 | 250 | 37 |
| 96 | 80 | 173 | 110 | 251 | 420 |
| 97 | 33 | 174 | 44 | 252 | 190 |
| 99 | 64 | 175 | 8 | 253 | 1500 |
| 101 | 24 | 176 | 29 | 254 | 84 |
| 103 | 27 | 177 | 30 | 255 | 500 |
| 104 | 2 | 178 | 16 | 256 | 170 |
| 105 | 64 | 179 | 10 | 257 | 540 |
| 106 | 56 | 180 | 7 | 258 | 190 |
| 107 | 29 | 181 | 4 | 259 | 430 |
| 108 | 76 | 182 | 4 | 260 | 130 |
| 109 | 24 | 183 | 10 | 261 | 110 |
| 110 | 150 | 184 | 170 | 262 | 2100 |
| 111 | 20 | 185 | 140 | 263 | 38 |
| 112 | | 186 | 23 | 264 | 31 |
| 113 | 6 | 187 | 4 | 265 | 2 |
| 114 | 7 | 188 | 4 | 266 | 59 |
| 115 | 10 | 189 | 3 | 267 | 16 |
| 116 | 20 | 190 | 140 | 268 | 18 |

**Tabelle 2:**

| **Beispiel Nr.** | **Hamster Chymase IC 50 [nM]** | **Beispiel Nr.** | **Hamster Chymase IC 50 [nM]** | **Beispiel Nr.** | **Hamster Chymase IC 50 [nM]** |
|---|---|---|---|---|---|
| 269 | 14 | 281 | 1 | 293 | 12 |
| 270 | 6 | 282 | 88 | 294 | 2 |
| 271 | 23 | 283 | 40 | 295 | 8 |
| 272 | 11 | 284 | 11 | 296 | 3 |
| 273 | 1100 | 285 | 42 | 297 | |
| 274 | 2 | 286 | 37 | 298 | 6 |
| 275 | 2300 | 287 | 4500 | 299 | 120 |
| 276 | 4 | 288 | 14 | 300 | 2 |
| 277 | 2 | 289 | 970 | 301 | 33 |
| 278 | 5 | 290 | 8 | 302 | 19 |
| 279 | 4 | 291 | 4 | 303 | 9 |
| 280 | 250 | 292 | | | |

### Rektovaginales Hamsterendometriose-Fibrose Modell

Zur Untersuchung der Wirkung der Testsubstanzen auf durch eine Endometriose vermittelte Fibrose bzw. die dadurch entstehenden Adhäsionen werden weibliche Syrische Hamster (ca. 150 g) im Alter von 3-4 Monaten eingesetzt. Der Zyklus der Tiere wird über eine Vaginalabstrichnadel bestimmt. Der Östrus wird bei den Tieren durch das Abfließen eines eiterähnlichen Sekretes aus der Scheide definiert. Am Tag nach dem Östrus erfolgt die Operation zur Induktion der rektovaginalen Endometriose und der Generierung der damit verbundenen Adhäsionen, Fibrosen und Fisteln. Eine rektovaginale Endometriose wird in den Tieren wie folgt induziert: Einen Tag nach dem Östrus werden die Tiere mit Isofluran narkotisiert und die Bauchdecke eröffnet.. Ca.1/3 des Uterus wird entnommen und bei dem entnommenen Stück das Endometrium vom Myometrium getrennt. Es werden Stanzen vom Endometrium mit dem Durchmesser von 5 mm genommen und diese mit jeweils 3 Stichen vernäht: Dabei wird das Peritoneum in den rektovaginalen Beutel und zwischen Blase und Uterus mit dem Uterus vernäht. Anschließend wird die Bauchdecke durch Setzen einer Naht verschlossen. Dadurch wird eine Endometriose mit generiert durch die sich innerhalb von 21 Tagen Adhäsionen ausbilden.
Beginnend am Tag der OP werden die Tiere täglich über einen Zeitraum von 21 Tagen mit der Testsubstanz behandelt. Die Testsubstanz wird oral in dem Vehikel Ethanol, Solutol, Wasser (v/v/v = 1/4/5) mit 2 ml/kg und den Dosierungen 3 mg/kg und 10 mg/kg einmal täglich appliziert. Die Krankheitsschwere wird basierend auf Adhäsionen und Fistelentwicklung nach Sektion der Tiere an Tag 21 bestimmt. wobei folgendes Scoring System angewendet wird:
0 = Keine Verwachsung, Blase frei bewegbar, keine Verbindung mit der Läsion, keine Fibrose und Adhäsion,
1 = Leichte Verwachsung, Blase nicht frei beweglich, Uterus bewegt sich durch die Verbindung über die Läsion leicht mit, Blase leicht mit der Läsion verwachsen und / oder geringe Adhäsion von der Läsion mit dem Umgebungsgewebe, Adhäsion sehr fein und durchscheinend,
2 = Mittlere Verwachsung, Blase nicht frei beweglich, Uterus bewegt sich durch die Verbindung über die Läsion mit, Blase ca. zur Hälfte mit der Läsion verwachsen und / oder Adhäsion von der Läsion mit dem Umgebungsgewebe,
3 = Starke Verwachsung, Blase nicht frei beweglich, Uterus bewegt sich durch die Verbindung über die Läsion stark mit, Blase größtenteils mit der Läsion verwachsen und / oder starke Adhäsion von der Läsion mit dem Umgebungsgewebe.

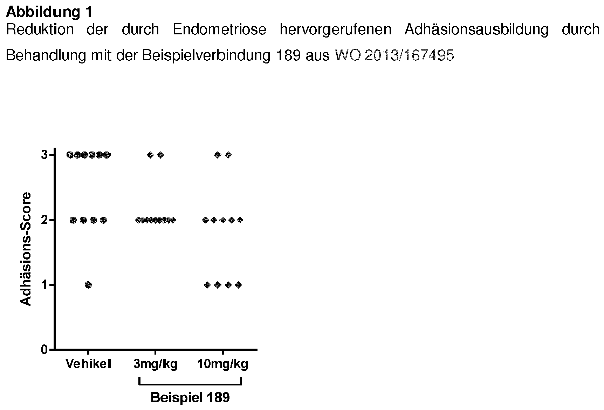

Abbildung 1 zeigt deutlich eine Reduktion durch die Testsubstanz Beispielverbindung 189 der durch Fibrose bedingten Adhäsionen, die durch eine induzierte Endometriose hervorgerufen wurden.

### Hamster-Adhäsions-Modell

Zur Untersuchung der Wirkung der Testsubstanzen auf eine postoperative Fibrose und/ oder postoperative Adhesionen und Fisteln werden weibliche Syrische Hamster (je ca. 150 g) im Alter von 3-4 Monaten eingesetzt. Der Zyklus der Tiere wird über eine Vaginalabstrichnadel bestimmt. Während des Östrus kann ein eiterähnliches Sekret aus der Scheide abfließen. Am Tag des Östrus erfolgt die Operation zur Generierung der Adhäsionen. Die Tiere werden dazu mit Isofluran narkotisiert und die Bauchdecke wird eröffnet. Vier ischämische Knoten werden jeweils lateral an der Peritonealwand durch basales Abbinden einer im Durchmesser ca. 5 mm großen Falte mit einem 4-0 Seiden-Nahtfaden gesetzt. Der Abstand zwischen den Knoten beträgt jeweils ca. 1 cm. Anschließend wird die Bauchdecke durch Setzen einer Naht verschlossen. Beginnend mit dem Tag der OP werden die Tiere werden täglich für 7 Tage mit der Testsubstanz behandelt. Die Testsubstanz wird oral in dem Vehikel Ethanol, Solutol, Wasser (v/v/v = 1/4/5) mit 2 ml/kg und den Dosierungen 1 mg/kg, 3 mg/kg und 10 mg/kg einmal täglich appliziert. Am Tag 7 erfolgt das Töten durch Dekapitieren oder unter tiefer Isoflurannarkose und anschließend die Autopsie. Die Anzahl der Adhäsionen pro ischämischen Knoten sowie ihre Größe und Beschaffenheit werden bestimmt, bevor das Gewebe für eine histologische und molekularbiologische Analyse entnommen wird. Außerdem wird Gewebe aus dem umliegenden Peritoneum als Kontrollgewebe konserviert. Die Krankheitsschwere wird basierend auf Adhäsionen und Fistelentwicklungnach Sektion bestimmt. Das Scoring wird für jeden ischämischen Knoten einzeln vorgenommen, wobei folgendes Scoring System angewendet wird:.
0 = Es befindet sich keine Adhäsion am ischämischen Knoten,
1 = Es befindet sich eine Adhäsion mit einem Blutgefäss am Knoten,
2 = Es befindet sich mehr als eine Adhäsion an dem Knoten,
3 = Es befinden sich viele Adhäsionen am Knoten, dieser ist aber gerade noch zu sehen.

Pro Tier wird dann der Durchschnitt der Scores aller Knoten ermittelt.

## Patentansprüche

1. Verwendung von Verbindungen der Formel (I) 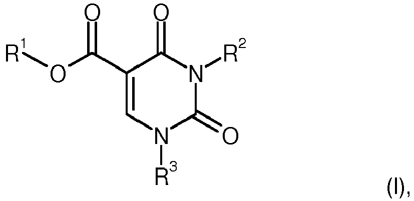 in welcher
R¹ für Wasserstoff oder (C₁-C₄)-Alkyl steht,
R² für eine Gruppe der Formel 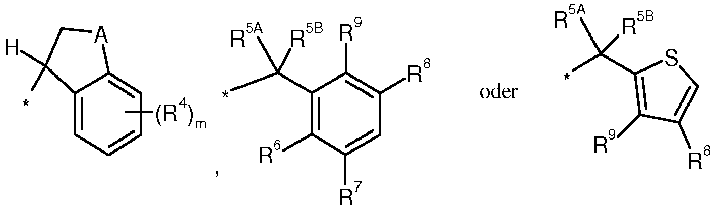 steht, wobei
* für die Anknüpfungsstelle an das Uracil-Stickstoffatom steht,
A für -CH₂-, -CH₂-CH₂-, -O-CH₂-## oder Sauerstoff steht,
worin
## für die Anknüpfstelle an den Phenyl-Ring steht,
m für eine Zahl 0, 1 oder 2 steht,
R⁴ für Halogen, Difluormethyl, Trifluormethyl, (C₁-C₄)-Alkyl, Difluormethoxy, Trifluormethoxy oder (C₁-C₄)-Alkoxy steht,
R^{5A} für Wasserstoff oder Deuterium steht,
R^{5B} für Wasserstoff, Deuterium oder (C₁-C₄)-Alkyl steht,
R⁶ für Wasserstoff oder Fluor steht,
R⁷ für Wasserstoff oder Fluor steht,
R⁸ für Halogen, Difluormethyl, Trifluormethyl, (C₁-C₄)-Alkyl oder Nitro steht,
R⁹ für Wasserstoff, Halogen, Difluormethyl, Trifluormethyl, (C₁-C₄)-Alkyl, Nitro oder (C₁-C₄)-Alkylthio steht,
R³ für eine Gruppe der Formel 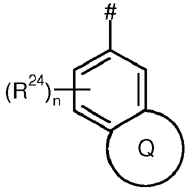 steht, wobei
# für die Anknüpfungsstelle an das Uracil-Stickstoffatom steht,
der Ring Q für 5- bis 7-gliedriges Heterocyclyl oder 5- oder 6-gliedriges Heteroaryl steht,
worin 5- bis 7-gliedriges Heterocyclyl und 5- oder 6-gliedriges Heteroaryl mit 1 bis 4 Substituenten unabhängig voneinander ausgewählt aus der Gruppe Halogen, Difluormethyl, Trifluormethyl, Trideuteromethyl, (C₁-C₆)-Alkyl, (C₃-C₇)-Cycloalkyl, Oxo, Hydroxy, (C₁-C₄)-Alkylcarbonyl, (C₁-C₄)-Alkoxycarbonyl, Aminocarbonyl und (C₁-C₄)-Alkylsulfonyl substituiert sein können,
worin (C₁-C₆)-Alkyl und (C₃-C₇)-Cycloalkyl ihrerseits mit 1 bis 3 Substituenten unabhängig voneinander ausgewählt aus der Gruppe Halogen, Cyano, Trifluormethyl, (C₃-C₇)-Cycloalkyl, Hydroxy, (C₁-C₄)-Alkoxy und 4- bis 7-gliedriges Heterocyclyl substituiert sein können,
und
worin zwei an ein Kohlenstoffatom von 5- bis 7-gliedriges Heterocyclyl und 5- oder 6-gliedriges Heteroaryl gebundene (C₁-C₆)-Alkyl-Reste zusammen mit dem Kohlenstoffatom, an das sie gebunden sind, einen 3- bis 6-gliedrigen Carboncyclus bilden können,
R²⁴ für Halogen, (C₁-C₄)-Alkyl oder (C₁-C₄)-Alkoxy steht,
n für eine Zahl 0, 1, 2 oder 3 steht,
sowie ihrer Salze, Solvate und Solvate der Salze zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.

2. Verwendung von Verbindungen der Formel (I) nach Anspruch 1, in welcher
R¹ für Wasserstoff, Methyl oder Ethyl steht,
R² für eine Gruppe der Formel 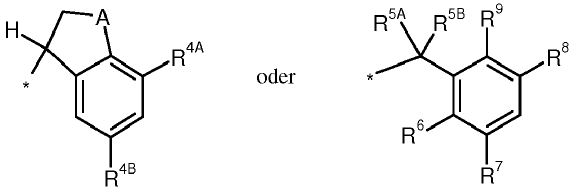 steht, wobei
* für die Anknüpfungsstelle an das Uracil-Stickstoffatom steht,
A für -CH₂-, -CH₂-CH₂-, -O-CH₂-## oder Sauerstoff steht,
worin
## für die Anknüpfstelle an den Phenyl-Ring steht,
R^{4A} für Wasserstoff, Fluor, Chlor, Trifluormethyl oder Methyl steht,
R^{4B} für Wasserstoff, Fluor, Chlor, Trifluormethyl oder Methyl steht,
mit der Massgabe, dass mindestens einer der Reste R^{4A} und R^{4B} von Wasserstoff verschieden ist,
R^{5A} für Wasserstoff steht,
R^{5B} für Wasserstoff steht,
R⁶ für Wasserstoff steht,
R⁷ für Wasserstoff steht,
R⁸ für Fluor, Chlor, Difluormethyl, Trifluormethyl oder Methyl steht,
R⁹ für Fluor, Chlor, Difluormethyl, Trifluormethyl oder Methyl steht,
R³ für eine Gruppe der Formel 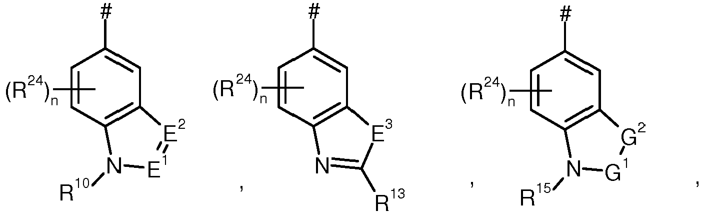 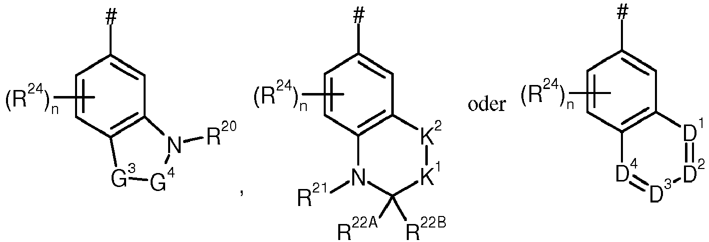 steht, wobei
# für die Anknüpfungsstelle an das Uracil-Stickstoffatom steht,
E¹ für CR¹¹ oder N steht,
worin
R¹¹ für Wasserstoff, (C₁-C₄)-Alkyl, (C₃-C₇)-Cycloalkyl oder Aminocarbonyl steht,
E² für CR¹² oder N steht,
worin
R¹² für Wasserstoff, (C₁-C₄)-Alkyl oder (C₃-C₇)-Cycloalkyl steht,
E³ für NR¹⁴ oder S steht,
worin
R¹⁴ für Wasserstoff, (C₁-C₄)-Alkyl oder (C₃-C₇)-Cycloalkyl steht,
G¹ für C=O oder SO₂ steht,
G² für CR^{16A}R^{16B}, NR¹⁷, O oder S steht,
worin
R^{16A} für Wasserstoff, Fluor, (C₁-C₄)-Alkyl oder Hydroxy steht,
R^{16B} für Wasserstoff, Fluor, Chlor, (C₁-C₄)-Alkyl oder Trifluormethyl steht,
oder
R^{16A} und R^{16B} bilden zusammen mit dem Kohlenstoffatom, an das sie gebunden sind, einen 3- bis 6-gliedrigen Carbocyclus,
R¹¹ für Wasserstoff, (C₁-C₆)-Alkyl, (C₃-C₇)-Cycloalkyl oder (C₁-C₄)-Alkoxycarbonyl steht,
worin (C₁-C₆)-Alkyl mit 1 bis 3 Subsituenten unabhängig voneinander ausgewählt aus der Gruppe Fluor, Trifluormethyl, Cyano, (C₃-C₇)-Cycloalkyl, Hydroxy, Trifluormethoxy, (C₁-C₄)-Alkoxy, Azetidinyl, Oxetanyl, Tetrahydrofuranyl und Pyrrolidinyl substituiert sein kann,
G³ für CR^{18A}R^{18B}, NR¹⁹, O oder S steht,
worin
R^{18A} für Wasserstoff, Fluor, (C₁-C₄)-Alkyl oder Hydroxy steht,
R^{18B} für Wasserstoff, Fluor, Chlor, (C₁-C₄)-Alkyl oder Trifluormethyl steht,
oder
R^{18A} und R^{18B} zusammen mit dem Kohlenstoffatom, an das sie gebunden sind, einen 3- bis 6-gliedrigen Carbocyclus bilden,
R¹⁹ für Wasserstoff, (C₁-C₆)-Alkyl, (C₃-C₇)-Cycloalkyl oder (C₁-C₄)-Alkoxycarbonyl steht,
worin (C₁-C₆)-Alkyl mit 1 bis 3 Subsituenten unabhängig voneinander ausgewählt aus der Gruppe Fluor, Trifluormethyl, Cyano, (C₃-C₇)-Cycloalkyl, Hydroxy, Trifluormethoxy, (C₁-C₄)-Alkoxy, Azetidinyl, Oxetanyl, Tetrahydrofuranyl und Pyrrolidinyl substituiert sein kann,
G⁴ für CH₂, C=O oder SO₂ steht,
K¹ für CH₂ oder O steht,
K² für CH₂ oder O steht,
mit der Massgabe, dass nur eine der Gruppe K¹ und K² für O steht,
D¹, D², D³ und D⁴ unanhängig voneinander für CR²³ oder N stehen,
worin
R²³ für Wasserstoff, Halogen, (C₁-C₆)-Alkyl oder (C₃-C₇)-Cycloalkyl steht,
mit der Maßgabe, dass maximal 2 der Gruppen D¹, D², D³ und D⁴ für N stehen,
R²⁴ für Fluor oder Methyl steht,
n für eine Zahl 0 oder 1 steht,
R¹⁰ für (C₁-C₄)-Alkyl oder (C₃-C₇)-Cycloalkyl steht,
worin (C₁-C₄)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R¹³ für Wasserstoff, (C₁-C₄)-Alkyl oder (C₃-C₇)-Cycloalkyl steht,
R¹⁵ für Wasserstoff, (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R²⁰ für Wasserstoff, (C₁-C₆)-Alkyl, (C₃-C₇)-Cycloalkyl oder (C₁-C₄)-Alkylcarbonyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R²¹ für Wasserstoff, (C₁-C₆)-Alkyl, (C₃-C₇)-Cycloalkyl oder (C₁-C₄)-Alkylsulfonyl steht,
R^{22A} für Wasserstoff oder (C₁-C₄)-Alkyl steht,
R^{22B} für Wasserstoff oder (C₁-C₄)-Alkyl steht,
oder
R^{22A} und R^{22B} bilden zusammen mit dem Kohlenstoffatom, an das sie gebunden sind, eine Carbonylgruppe,
sowie ihrer Salze, Solvate und Solvate der Salze zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.

3. Verwendung von Verbindungen der Formel (I) nach Anspruch 1 oder 2, in welcher
R¹ für Wasserstoff steht,
R² für eine Gruppe der Formel 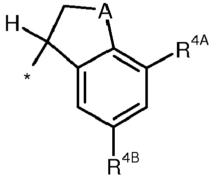 steht, wobei
* für die Anknüpfungsstelle an das Uracil-Stickstoffatom steht,
A für -CH₂- steht,
R^{4A} für Chlor oder Trifluormethyl steht,
R^{4B} für Wasserstoff steht,
R³ für eine Gruppe der Formel 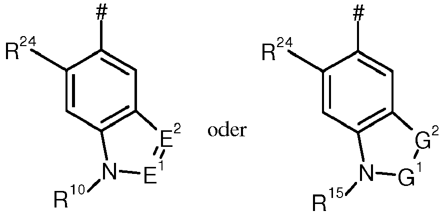 steht, wobei
# für die Anknüpfungsstelle an das Uracil-Stickstoffatom steht,
E¹ für CR¹¹ steht,
worin
R¹¹ für Wasserstoff steht,
E² für N steht,
G¹ für C=O steht,
G² für CR^{16A}R^{16B}, NR¹⁷, O oder S steht,
worin
R^{16A} für Wasserstoff, Fluor, Methyl oder Hydroxy steht,
R^{16B} für Wasserstoff, Fluor, Methyl oder Trifluormethyl steht,
oder
R^{16A} und R^{16B} zusammen mit dem Kohlenstoffatom, an das sie gebunden sind, einen Cyclopropyl-Ring bilden,
R¹⁷ für Wasserstoff, (C₁-C₄)-Alkyl oder (C₃-C₅)-Cycloalkyl steht, worin (C₁-C₄)-Alkyl mit 1 bis 3 Substituenten unabhängig voneinander ausgewählt aus der Gruppe Fluor, Trifluormethyl, Cyano, Cyclopropyl, Cyclobutyl, Hydroxy, Trifluormethoxy, Methoxy, Ethoxy, Azetidinyl, Oxetanyl, Tetrahydrofuranyl und Pyrrolidinyl substituiert sein kann,
R²⁴ für Wasserstoff oder Fluor steht,
R¹⁰ für (C₁-C₄)-Alkyl steht,
R¹⁵ für Wasserstoff, Methyl oder Ethyl steht, worin Methyl und Ethyl mit 1 Substituenten ausgewählt aus der Gruppe Fluor, Trifluormethyl und Cyclopropyl substituiert sein können,
sowie ihrer Salze, Solvate und Solvate der Salze zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.

4. Verwendung von Verbindungen der Formel (I) nach Anspruch 1 oder 2, in welcher
R¹ für Wasserstoff steht,
R² für eine Gruppe der Formel 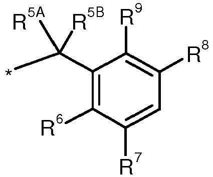 steht, wobei
* für die Anknüpfungsstelle an das Uracil-Stickstoffatom steht,
R^{5A} für Wasserstoff steht,
R^{5B} für Wasserstoff steht,
R⁶ für Wasserstoff steht,
R⁷ für Wasserstoff steht,
R⁸ für Fluor, Chlor oder Trifluormethyl steht,
R⁹ für Fluor, Chlor, Trifluormethyl oder Methyl steht,
R³ für eine Gruppe der Formel 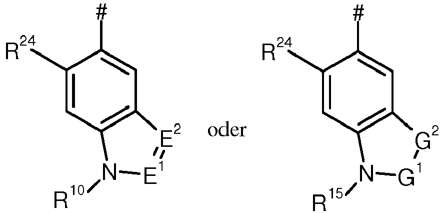 steht, wobei
# für die Anknüpfungsstelle an das Uracil-Stickstoffatom steht,
E¹ für CR¹¹ steht,
worin
R¹¹ für Wasserstoff steht,
E² für N steht,
G¹ für C=O steht,
G² für CR^{16A}R^{16B}, NR¹⁷, O oder S steht,
worin
R^{16A} für Wasserstoff, Fluor, Methyl oder Hydroxy steht,
R^{16B} für Wasserstoff, Fluor, Methyl oder Trifluormethyl steht,
oder
R^{16A} und R^{16B} zusammen mit dem Kohlenstoffatom, an das sie gebunden sind, einen Cyclopropyl-Ring bilden,
R¹⁷ für Wasserstoff, (C₁-C₄)-Alkyl oder (C₃-C₅)-Cycloalkyl steht,
worin (C₁-C₄)-Alkyl mit 1 bis 3 Substituenten unabhängig voneinander ausgewählt aus der Gruppe Fluor, Trifluormethyl, Cyano, Cyclopropyl, Cyclobutyl, Hydroxy, Trifluormethoxy, Methoxy, Ethoxy, Azetidinyl, Oxetanyl, Tetrahydrofuranyl und Pyrrolidinyl substituiert sein kann,
R²⁴ für Wasserstoff oder Fluor steht,
R¹⁰ für (C₁-C₄)-Alkyl steht,
R¹⁵ für Wasserstoff, Methyl oder Ethyl steht, worin Methyl und Ethyl mit 1 Substituenten ausgewählt aus der Gruppe Fluor, Trifluormethyl und Cyclopropyl substituiert sein können,
sowie ihrer Salze, Solvate und Solvate der Salze zur Behandlung und Prävention der Endometriose, der Endometriose-assoziierten Fibrose, der Adenomyose, des mit einer Endometrioseerkrankung assoziierten Schmerzes und der Behandlung und Prävention von postoperativen Fibrosen und Adhäsionen.

5. Verwendung von Verbindungen der Formel (I) nach einem der vorangegangenen Ansprüche zur Behandlung und/oder Prophylaxe von chronischen Schmerzen im kleinen Becken, der nicht mit Endometriose assoziiert ist.
